# EUROPEAN PATENT SPECIFICATION

(11) **EP 3 417 294 B1**
(45) Date of publication and mention of the grant of the patent: **01.12.2021**
(21) Application number: 17753704.0
(22) Date of filing: 14.02.2017
(51) Int. Cl.: C07K 16/28, G01N 33/574, A61K 38/00, A61P 35/00, A61K 31/502, A61K 31/506, A61K 45/06, A61K 48/00, A61P 9/10, A61P 43/00, A61K 39/395

(54) **METHODS COMPRISING FIXED INTERMITTENT DOSING OF CEDIRANIB**
VERFAHREN MIT FIXER INTERMITTIERENDER DOSIERUNG VON CEDIRANIB
PROCÉDÉS COMPRENANT UN DOSAGE INTERMITTENT ET FIXE DE CEDIRANIB

(30) Priority: 15.02.2016 US 201662295421 P
(43) Date of publication of application: 26.12.2018
(73) Proprietor: Astrazeneca AB, 151 85 Södertälje (SE); The United States of America, as Represented By the Secretary, Department of Health and Human Services, Bethesda, MD 20892-7660 (US)
(72) Inventor: BARRY, Simon, Cambridge CB4 0WG (GB); KENDREW, Jane, Macclesfield Cheshire SK10 4TG (GB); HO, Tony, Gaithersburg, MD 20878 (US); WEDGE, Stephen, Robert, Granta Park Cambridge CB21 6GH (GB); IVY,Susan Percy, Bethesda, MD 20892-7660 (US); KOHN, Elise, Bethesda, MD 20892-7660 (US); LEE, Jung-Min, Bethesda, MD 20892-7660 (US)
(74) Representative: Finnegan Europe LLP
(86) International application number: PCT/US2017/017804
(87) International publication number: WO 2017/142871

(56) References cited:
- WO-A1-2006/035203
- US-A1- 2014 377 285
- JOYCE F LIU ET AL: "A randomised phase 2 study of combination cediranib and olaparib versus olaparib alone as recurrence therapy in platinum-sensitive ovarian cancer.", THE LANCET ONCOLOGY, vol. 15, no. 11, 1 October 2014 (2014-10-01), pages 1207-1214, XP055490379, AMSTERDAM, NL ISSN: 1470-2045, DOI: 10.1016/S1470-2045(14)70391-2
- KIERAN MARK W ET AL: "A phase I trial and PK study of cediranib (AZD2171), an orally bioavailable pan-VEGFR inhibitor, in children with recurrent or refractory primary CNS tumors", CHILD'S NERVOUS SYSTEM, vol. 31, no. 9, 19 July 2015 (2015-07-19), pages 1433-1445, XP035541345, SPRINGER, BERLIN, DE ISSN: 0256-7040, DOI: 10.1007/S00381-015-2812-5 [retrieved on 2015-07-19]
- CHARLES J RYAN ET AL: "Phase I dose escalation and pharmacokinetic study of AZD2171, an inhibitor of the vascular endothelial growth factor receptor tyrosine kinase, in patients with hormone refractory prostate cancer (HRPC)", INVESTIGATIONAL NEW DRUGS ; THE JOURNAL OF NEW ANTICANCER AGENTS, vol. 25, no. 5, 26 April 2007 (2007-04-26) , pages 445-451, XP019526142, KLUWER ACADEMIC PUBLISHERS, BO ISSN: 1573-0646, DOI: 10.1007/S10637-007-9050-Y
- LEE JUNG-MIN ET AL: "Safety and Clinical Activity of the Programmed Death-Ligand 1 Inhibitor Durvalumab in Combination With Poly (ADP-Ribose) Polymerase Inhibitor Olaparib or Vascular Endothelial Growth Factor Receptor 1-3 Inhibitor Cediranib in Women's Cancers: A Dose-Escalation, Phase I Study.", JOURNAL OF CLINICAL ONCOLOGY : OFFICIAL JOURNAL OF THE AMERICAN SOCIETY OF CLINICAL ONCOLOGY, vol. 35, no. 19, 1 July 2017 (2017-07-01), pages 2193-2202+9pp, XP002792437, ISSN: 1527-7755
- LIU, JF et al.: "A Phase 1 trial of the PARP inhibitor olaparib (AZD2281) in combination with the anti-angiogenic cediranib (AZD2171) in recurrent epithelial ovarian or triple-negative breast cancer", European Journal of Cancer, vol. 49, no. 14, September 2013 (2013-09), pages 2972-2978, XP055410070,
- WEBSTER, RM: "Combination therapies in oncology", Nature Reviews., vol. 15, no. 2, 6 February 2016 (2016-02-06), pages 81-82, XP055410072,
- "Cediranib Maleate and Olaparib in Treating Patients With Recurrent Ovarian, Fallopian Tube", Peritoneal Cancer or Recurrent Triple-Negative Breast Cancer, October 2016 (2016-10), XP055575178, Retrieved from the Internet: URL:https://clinicaltrials.gov/ct2/show/st udy/NCT01116648 [retrieved on 2017-04-03]

## Description

Disclosed herein are repeating cycles of administration of a composition comprising cediranib according to a fixed intermittent dosing regimen for use in producing an antiangiogenic and/or vascular permeability reducing effect in a warm-blooded animal, such as a human being, in need thereof. Also disclosed herein are compositions comprising cediranib for use in treating at least one disease state associated with angiogenesis in a human being in need thereof which comprises repeating cycles of administration of a composition comprising cediranib according to a fixed intermittent dosing regimen. The fixed intermittent dosing regimens comprise administration of an effective amount of the composition comprising cediranib on one or more consecutive days of a cycle followed by one or more consecutive days of rest on which said composition is not administered. The compositions comprising cediranib may be used in monotherapy or may further be used in combination therapy with one or more partner drugs.

Repeating cycles of administration of a composition comprising cediranib according to a fixed intermittent dosing regimen are provided for use in reducing the total dose of cediranib required to provide effective VEGF inhibition as well as ways of reducing adverse events and toxicity due to cediranib administration.

The disclosed uses may maintain cover on the VEGF pathway despite reduction of total doses of cediranib, and thus also may provide uses for improving the overall therapeutic index of cediranib. Furthermore, these disclosures provide new ways of increasing repair of healthy, non-cancerous tissue during treatment of cancer using combination therapies.

Angiogenesis, the process of new blood vessel formation, plays an important role in a variety of processes including embryonic development, wound healing and several components of female reproductive function. Undesirable or pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacol. Sci. 16: 57-66; Folkman, 1995, Nature Medicine 1: 27-31). Indeed, angiogenesis is essential to tumor growth and metastasis. (Folkman J. Tumor angiogenesis: therapeutic implications. N. Engl. J. Med. 1971;285:1182-6; Cullinan-Bove et al, 1993, Endocrinology 133: 829-837; Senger et al, 1993, Cancer and Metastasis Reviews, 12: 303-324).

The inhibition of angiogenesis is therefore a possibility for the treatment of cancer. Disruption of blood vessel formation may be possible at several stages in the angiogenic process. Since vascular endothelial growth factor (VEGF) is known to be an important proangiogenic factor (Ferrara N. Molecular and biological properties of vascular endothelial growth factor. J. Mol. Med. 1999;77:527-43; Ferrara N. VEGF and the quest for tumor angiogenesis factors. Nature Reviews Cancer 2002;2:795-803.), VEGF and its receptor (VEGFR) are targets for inhibition of angiogenesis (Kim et al, 1993, Nature 362: 841-844).

Receptor tyrosine kinases (RTKs) are important in the transmission of biochemical signals across the plasma membrane of cells. These transmembrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity which leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified. One of these subfamilies is presently comprised by the fms-like tyrosine kinase receptor, Flt-1, the kinase insert domain-containing receptor, KDR (also referred to as Flk-1), and another fms-like tyrosine kinase receptor, Flt-4. Two of these related RTKs, Flt-1 and KDR, have been shown to bind VEGF with high affinity (De Vries et al, 1992, Science 255: 989-991; Terman et al, 1992, Biochem. Biophys. Res. Comm. 1992, 187: 1579-1586). Binding of VEGF to these receptors expressed in heterologous cells has been associated with changes in the tyrosine phosphorylation status of cellular proteins and calcium fluxes.

Compounds which inhibit the effects of VEGF are of value in the treatment of disease states associated with angiogenesis and/or increased vascular permeability such as cancer (including leukemia, multiple myeloma and lymphoma), diabetes, psoriasis, rheumatoid arthritis, Kaposi's sarcoma, haemangioma, acute and chronic nephropathies, atheroma, arterial restenosis, autoimmune diseases, acute inflammation, excessive scar formation and adhesions, endometriosis, lymphoedema, dysfunctional uterine bleeding and ocular diseases with retinal vessel proliferation including macular degeneration.

Two high-affinity receptors for VEGF with associated tyrosine kinase activity have been identified on human vascular endothelium: VEGFR-1 and VEGFR-2. VEGFR-3, a third member of the VEGFR gene family, plays a key role in the regulation of endothelial tip cells that initiate the formation of new blood vessels and is thought to be important for lymphangiogenesis. VEGFR-3 is activated by the ligands VEGF-C and VEGF-D and has potential to cross talk to VEGFR-2. Although their relative contributions in mediating tumor progression have not been resolved, some studies suggest VEGFR-2 may have a predominant role (Ferrara N. Molecular and biological properties of vascular endothelial growth factor. J. Mol. Med. 1999;77:527-43).

Liu et. al., Lancet Oncol., 2014, 15(11), 1207-1214 discloses a randomized phase 2 study of combination cediranib and olaparib versus olaparib alone as recurrence therapy in platinum-sensitive ovarian cancer.

Cediranib, as used herein, refers to a compound having IUPAC name of 4-[(4-fluoro-2-methyl-1H-indol-5-yl)oxy]-6-methoxy-7-[3-(pyrrolidin-1-yl)propoxy]quinazoline maleate, also referred to as AZD2171 maleate and has the following structure:

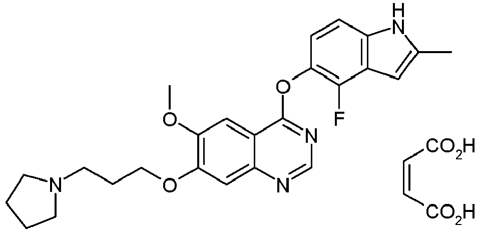

As used herein, cediranib includes its salts, esters, prodrugs, hydrates, and solvates.

The free base 4-[(4-fluoro-2-methyl-1H-indol-5-yl)oxy]-6-methoxy-7-[3-(pyrrolidin-1-yl)propoxy]quinazoline is exemplified in WO 00/47212 (and U.S. Patent No. 7,074,800) for example as Example 240. Its maleate salt, cediranib, is disclosed and exemplified in U.S. Patent No. 8,859,570.

Cediranib is an orally active VEGF receptor tyrosine kinase (RTK) inhibitor of all three VEGF receptors (VEGFR-1,-2,-3), which act as receptors for VEGF-A, B, C, and D. Targeting all three VEGFRs results in comprehensive inhibition of the VEGF signaling pathway. Inhibition of signalling through VEGFR-2 reduces angiogenesis, neovascular survival, and vascular permeability. Inhibition of signaling through VEGFR-3 additionally reduces lymphangiogenesis, contributing to a reduction in metastatic spread. Cediranib has been reported to inhibit the growth of tumors in a dose-dependent manner in a range of preclinical models, associated with reduction in microvessel density and metastasis. Collectively, these changes indicate that cediranib inhibits tumor growth, metastases, and vascular permeability through inhibition of the VEGFR family (Brave et al. Assessing the activity of cediranib, a VEGFR-2/3 tyrosine kinase inhibitor, against VEGFR-1 and members of the structurally related PDGFR family. Mol. Cancer Ther. 2011; 10(5):861-73; Heckman et al. The tyrosine kinase inhibitor cediranib blocks ligand-induced vascular endothelial growth factor receptor-3 activity and lymphangiogenesis. Cancer Res. 2008;68(12):4754-62; Smith et al. Acute pharmacodynamic and antivascular effects of the vascular endothelial growth factor signaling inhibitor AZD2171 in Calu-6 human lung tumor xenografts. Mol. Cancer Ther. 2007;6(8):2198-208; Wedge et al. AZD2171: A Highly Potent, Orally Bioavailable, Vascular Endothelial Growth Factor Receptor-2 Tyrosine Kinase Inhibitor for the Treatment of Cancer. Cancer Res. 2005; 65:4389-400.)

Cediranib has been evaluated in a broad clinical program that includes both monotherapy and combination therapy studies, in multiple tumor types, including for example colorectal cancer, glioblastoma, non-small cell lung cancer (NSCLC), small cell lung cancer (SCLC), renal cell carcinoma (RCC), alveolar soft part sarcoma (ASPS), and ovarian cancer, as well as a large number of signal searching studies in a range of other tumor types. The feasibility, activity, and pharmacokinetics (PK) of cediranib have been explored in combination with carboplatin and paclitaxel (Laurie et al. Phase I pharmacokinetic study of daily oral AZD2171, an inhibitor of vascular endothelial growth factor tyrosine kinases, in combination with carboplatin and paclitaxel in patients with advanced non-small cell lung cancer: the National Cancer Institute of Canada Clinical Trials Group. J. Clin. Oncol. 2008;26(11): 1871-78) and cisplatin and gemcitabine (Goss et al. Phase I pharmacokinetic study of daily oral cediranib, an inhibitor of vascular endothelial growth factor tyrosine kinases, in combination with cisplatin and gemcitabine in patients with advanced non-small cell lung cancer: a study of the National Cancer Institute of Canada Clinical Trials Group. Eur. J. Cancer 2009;45(5):782-8).

Disclosed herein are at least two repeating cycles of administration of a composition comprising cediranib according to a fixed intermittent dosing regimen for use in producing an antiangiogenic and/or vascular permeability reducing effect in a warm-blooded animal, such as a human being. Also disclosed herein are at least two repeating cycles of administration of a composition comprising cediranib according to a fixed intermittent dosing regimen for use in methods of treating at least one disease state associated with angiogenesis in a human being. The fixed intermittent dosing regimens comprise administration of an effective amount of the composition comprising cediranib on five consecutive days of a cycle, followed by two consecutive days of rest, on which said composition is not administered. These uses comprise only the use of compositions comprising cediranib and thus may be used in monotherapy or may further comprise the administration of one or more partner drugs and thus may be used in combination therapy.

Repeating cycles, such as at least two cycles, of administration of a composition comprising cediranib according to a fixed intermittent dosing regimen are useful for reducing the total dose of cediranib required to provide effective VEGF inhibition as well as reducing adverse events and/or toxicity due to cediranib administration. Non-limiting examples of adverse events and toxicity identified risks for cediranib include diarrhea, severe fatigue, severe neutropenia and febrile neutropenia, hypertension, GI perforation, fistulae, arterial thromboembolism and posterior reversible encephalopathy syndrome (PRES).

The disclosed uses may maintain cover on the VEGF pathway despite reduction of total doses of cediranib, and thus also may provide ways of improving the overall therapeutic index of cediranib. Furthermore, these disclosures provide new ways to increase repair of healthy, non-cancerous tissue during treatment of cancer using combination therapies.

The new uses comprising administration of a composition comprising cediranib according to a fixed intermittent dosing regimen may surprisingly result in maintenance of tumor control, unlike dosing schedules involving 7 days off from cediranib and also unlike previous studies involving unscheduled dose holidays from a continuous dosing regimen necessitated by adverse events in patients. The new uses thereby allow administration of a reduced total dose of cediranib required to provide effective VEGF inhibition. Accordingly, the use of the new dosing regimen also provides a means of reducing adverse events and/or toxicity due to cediranib administration while maintaining cover on the VEGF pathway. Furthermore, this presents a way of increasing repair of healthy, non-cancerous tissue during treatment of cancer using combination therapies.

### BRIEF DESCRIPTION OF THE DRAWINGS

Figures falling outside the scope of the appended claims are provided for reference purposes only.

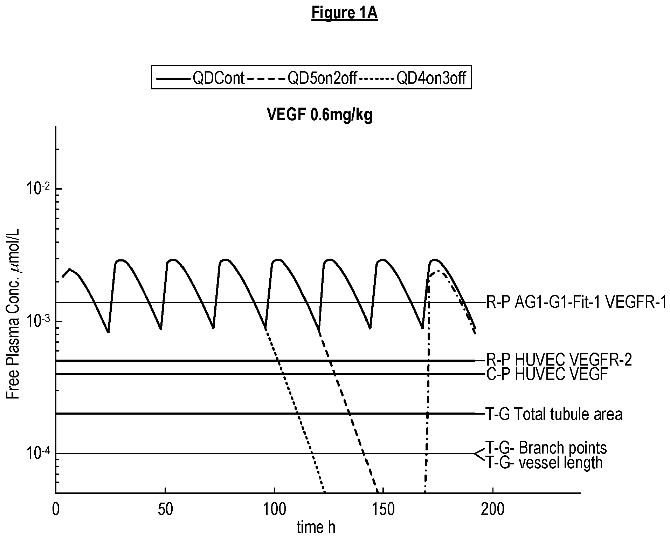

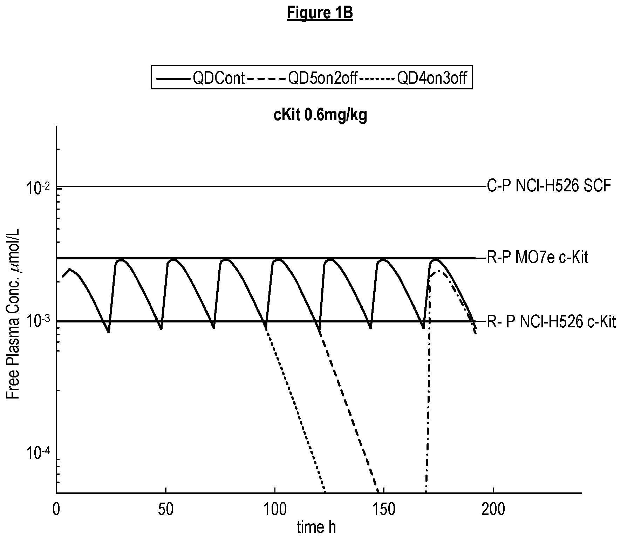

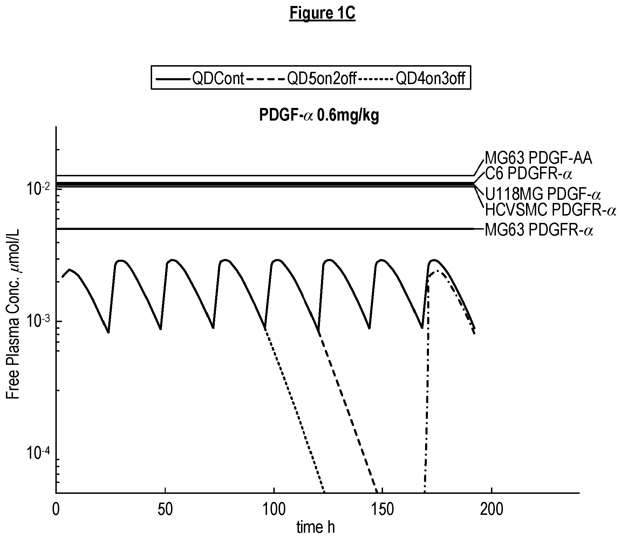

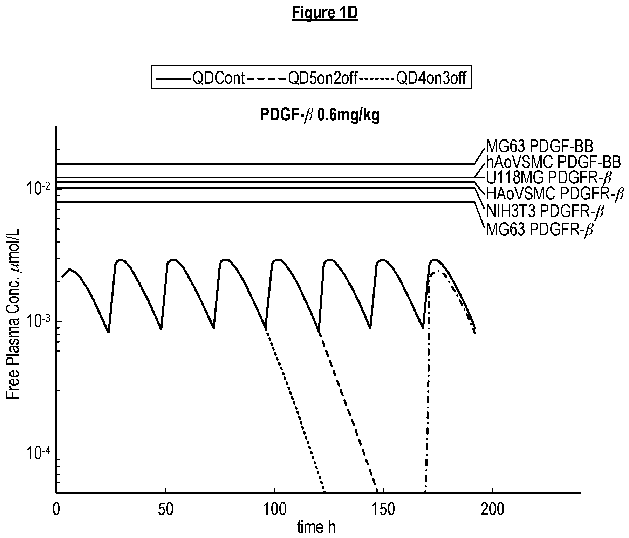

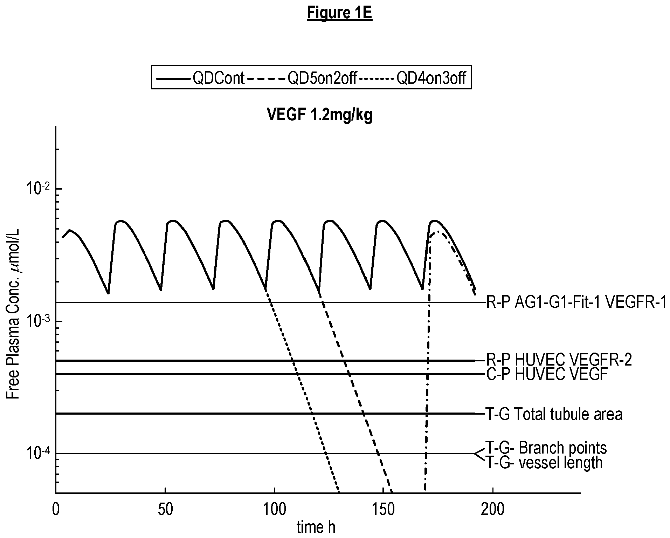

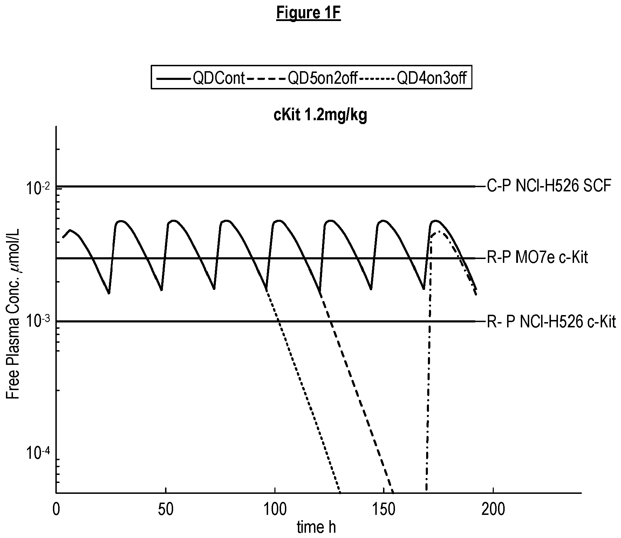

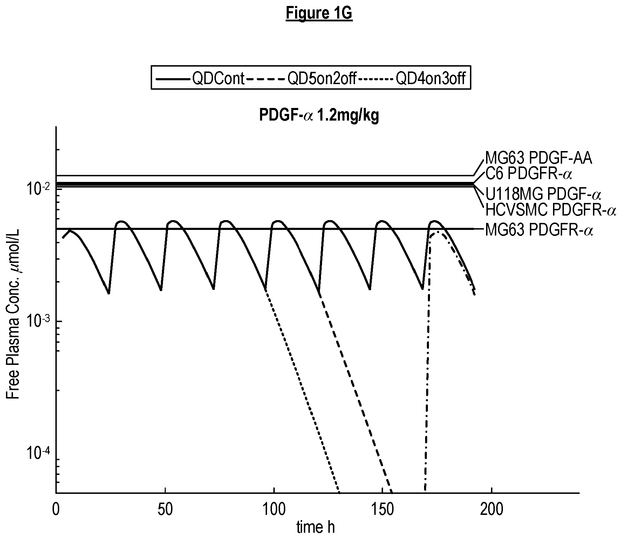

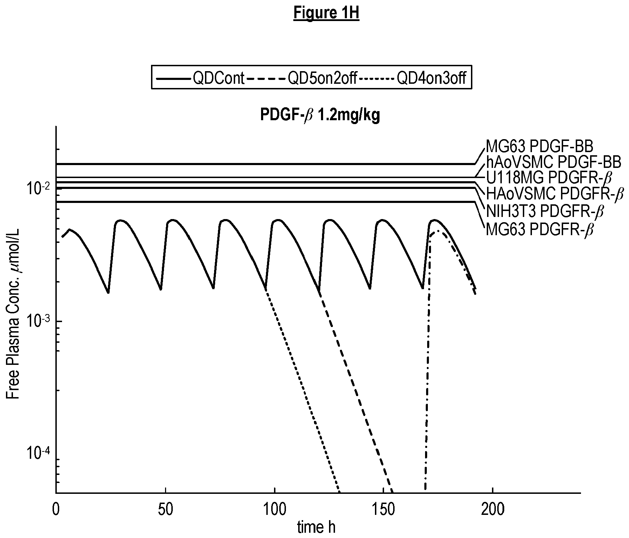

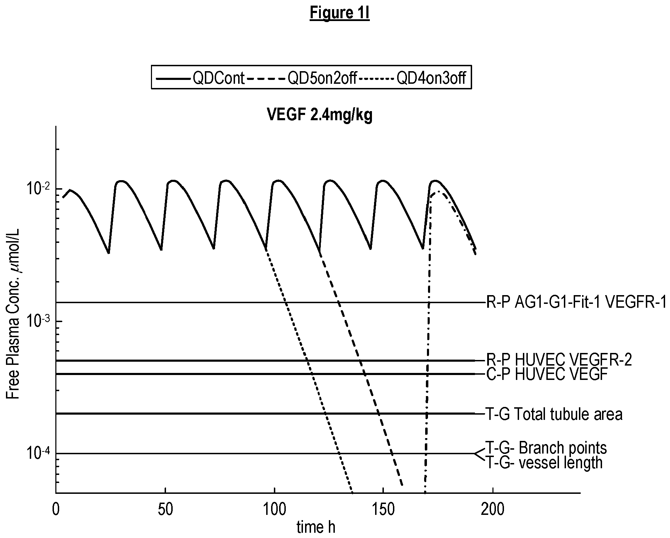

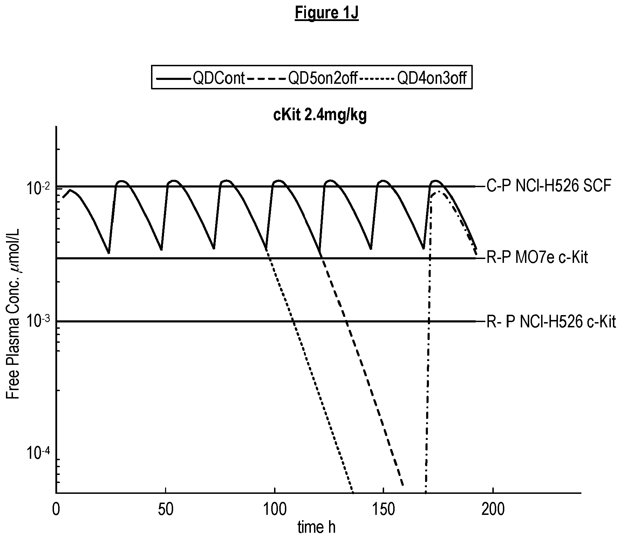

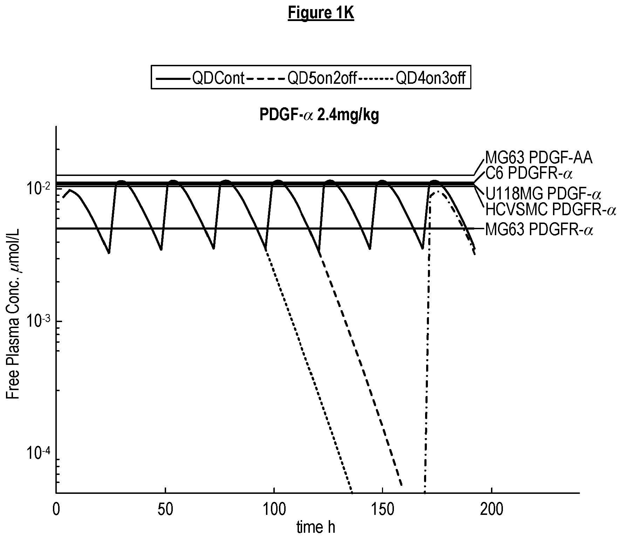

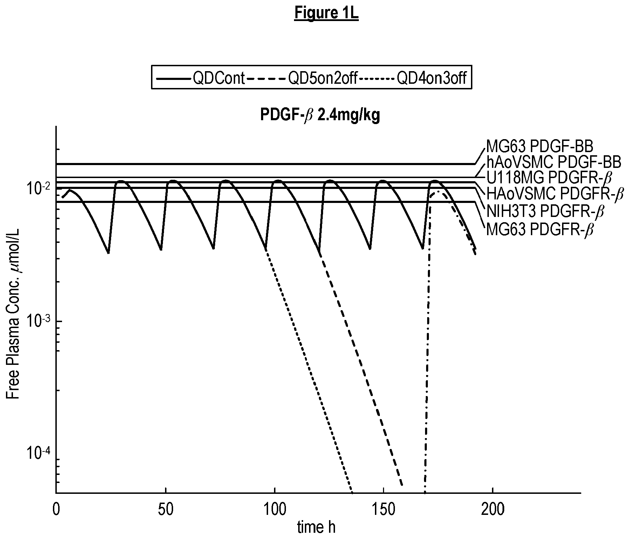

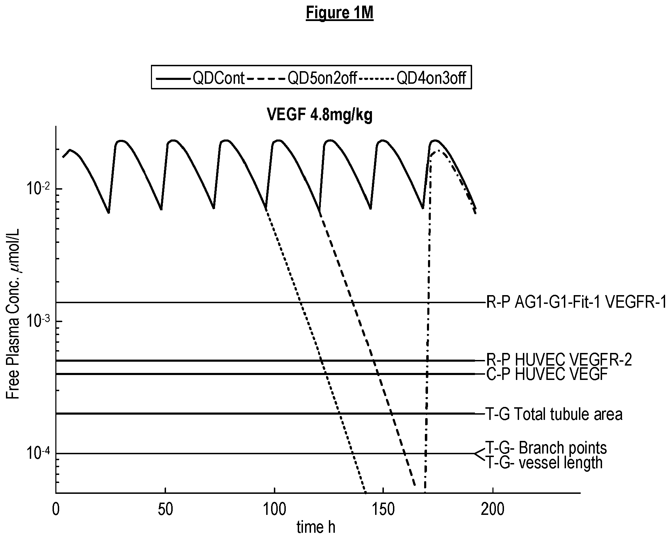

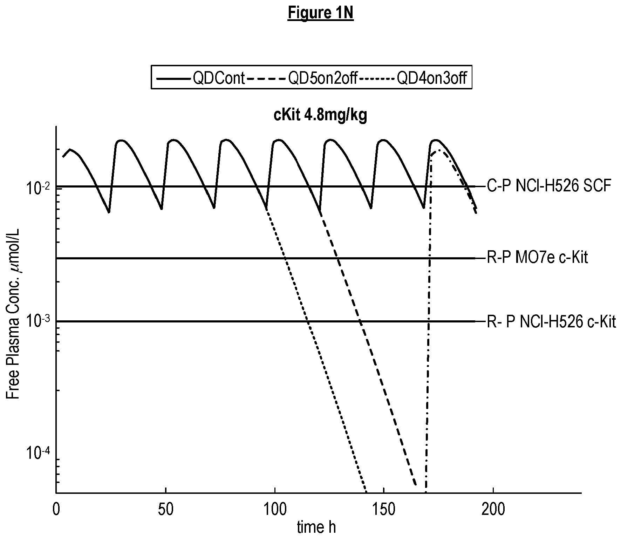

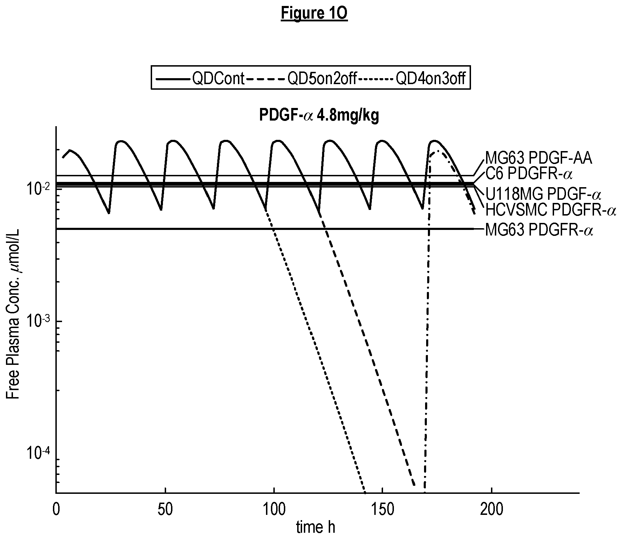

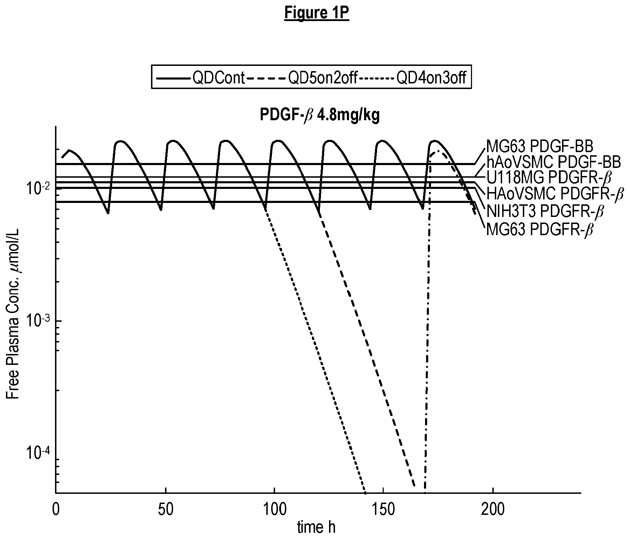

Figures 1(A)-(P) show modelled mean free plasma concentrations of cediranib over time in pre-clinical models dosed at a). 0.6 mg/kg (Figures 1A-1D), b). 1.2 mg/kg (Figures 1E-1H), c). 2.4 mg/kg (Figures 1I-L), or d). 4.8 mg/kg (Figures 1M-1P), respectively, following dosing regimens of once daily continuous (QDCont), once daily 5 days on 2 days off (QD5on2off), or once daily 4 days (QD4on3off), with horizontal lines showing the cellular ICso values generated in vitro for inhibition of pVEGFR, pKit, pPDGFRα, and pPDGFRβ as indicated. For the plots exemplifying VEGFR and Kit cover, R-P stands for receptor phosphorylation in a cell based assay; C-P stands for cell proliferation; for VEGFR plots, T-G stands for tubule growth in an endothelial-fibroblast co-culture assay. The relevant cell lines are indicated. For the graphs depicting PDGFRα and β cover, inhibition of PDGFRα and PDGFRβ phosphorylation and PDGFBB or PDGFAA driven proliferation are shown together with the relevant cell lines.

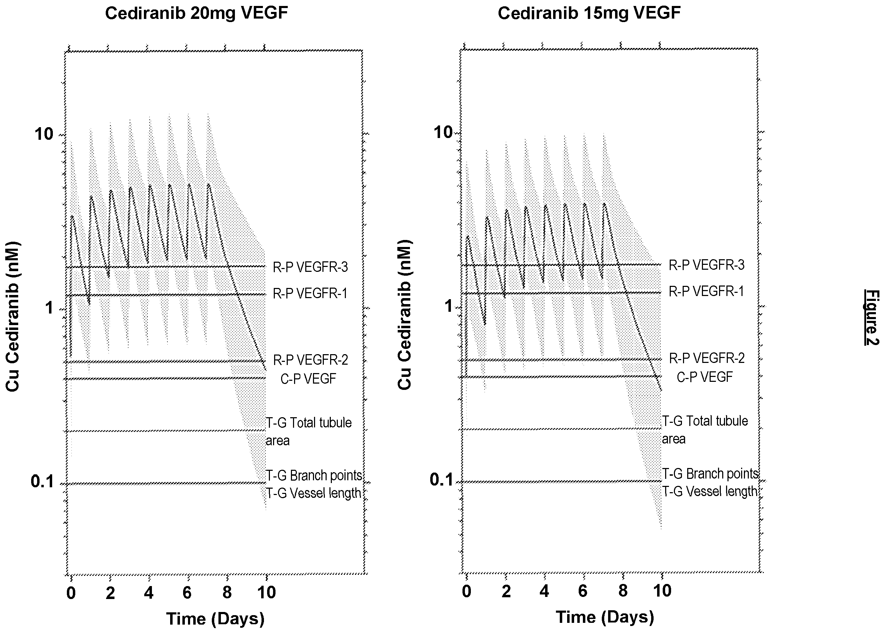

Figure 2 shows modelled mean the free drug exposure in humans for cediranib (black line) versus time for 20 mg and 15 mg doses of cediranib.

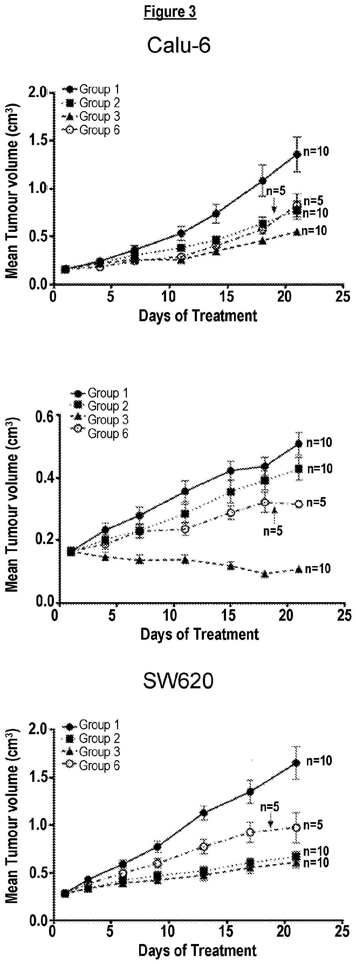

Figure 3 shows mean tumor volume versus time from three efficacy studies (performed in Calu6, A498, and SW620 tumor xenografts) each using four oral dosing regimen groups (Group 1 (Vehicle once daily for 21 days), Group 2 (1.5 mg/kg cediranib once daily for 21 days), Group 3 (3 mg/kg cediranib once daily for 21 days), and Group 6 (three 7-day cycles of 1.5 mg/kg cediranib once daily for 5 days followed by vehicle once daily for 2 days). Data are shown as the mean (+/- standard error of the mean). n shows the number of animals that were terminated at the relevant day.

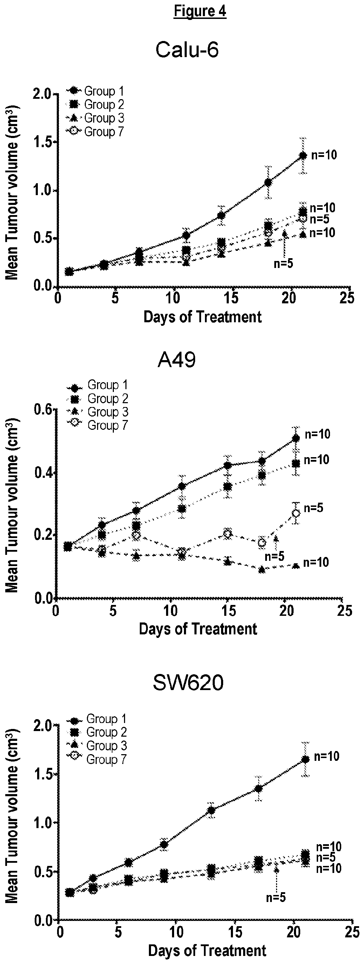

Figure 4 shows mean tumor volume versus time from three efficacy studies (in Calu6, A498, and SW620 tumor xenografts) each using four oral dosing regimen groups (Group 1 (Vehicle once daily for 21 days), Group 2 (1.5 mg/kg cediranib once daily for 21 days), Group 3 (3 mg/kg cediranib once daily for 21 days), and Group 7 (three 7-day cycles of 3 mg/kg cediranib once daily for 5 days followed by vehicle once daily for 2 days). Data are shown as the mean (+/- standard error of the mean). n shows the number of animals that were terminated at the relevant day.

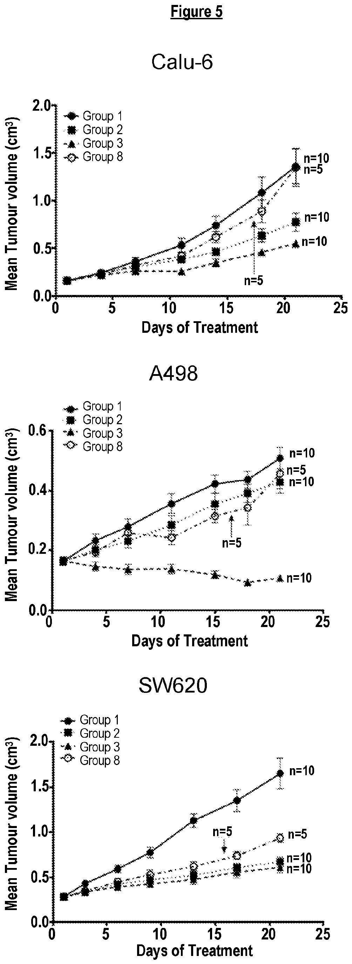

Figure 5 shows mean tumor volume versus time from three efficacy studies (Calu6, A498, and SW620) each using four oral dosing regimen groups (Group 1 (Vehicle once daily for 21 days), Group 2 (1.5 mg/kg cediranib once daily for 21 days), Group 3 (3 mg/kg cediranib once daily for 21 days), and Group 8 (three 7-day cycles of 1.5 mg/kg cediranib once daily for 3 days followed by vehicle once daily for 4 days). Data are shown as the mean (+/- standard error of the mean). n shows the number of animals that were terminated at the relevant day.

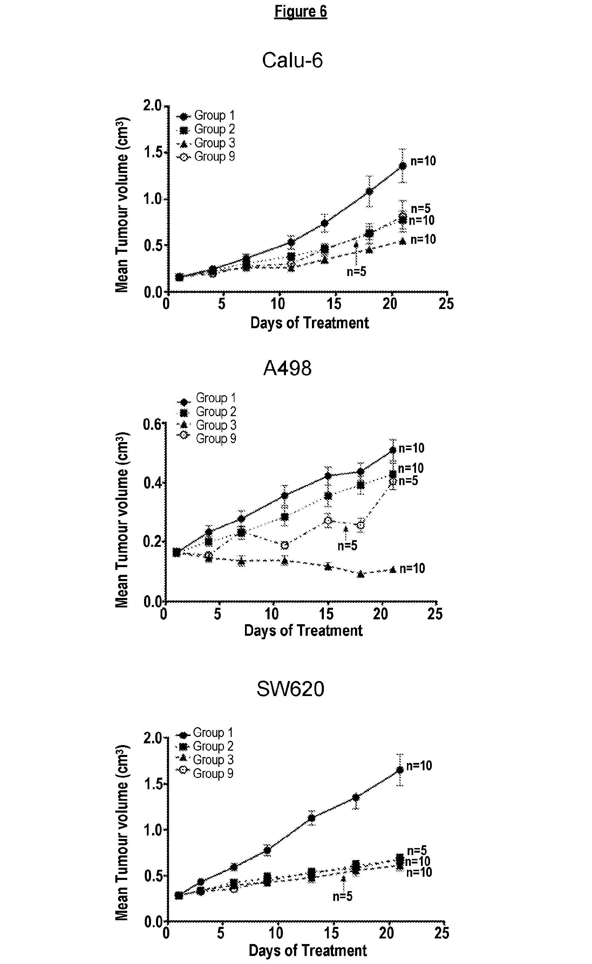

Figure 6 shows mean tumor volume versus time from three efficacy studies (Calu6, A498, and SW620) each using four oral dosing regimen groups (Group 1 (Vehicle once daily for 21 days), Group 2 (1.5 mg/kg cediranib once daily for 21 days), Group 3 (3 mg/kg cediranib once daily for 21 days), and Group 9 (three 7-day cycles of 3 mg/kg cediranib once daily for 3 days followed by vehicle once daily for 4 days). Data are shown as the mean (+/- standard error of the mean). n shows the number of animals that were terminated at the relevant day.

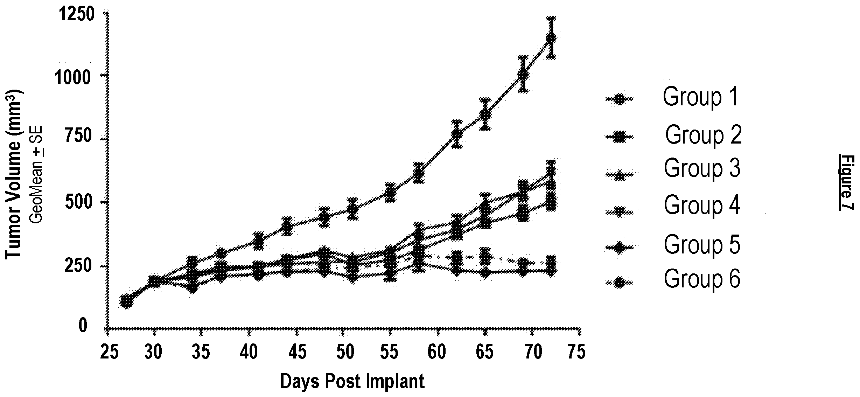

Figure 7 shows mean tumour volume versus time from an efficacy study performed in the OV2022 (ovarian cancer) patient derived tumour xenograft model. This study compared cediranib and olaparib combinations where cediranib was administered once daily or once daily on a 5 days on 2 days off schedule. Group 1 - control, Group 2 - cediranib 3mg/kg once daily, Group 3 - cediranib 3mg/kg once daily 5 days on 2 days off, Group 4 - olaparib 100mg/kg once daily, Group 5 - cediranib 3mg/kg once daily plus olaparib 100mg/kg once daily, Group 6 - cediranib 3mg/kg once daily 5 days on 2 days off plus olaparib 100mg/kg once daily.

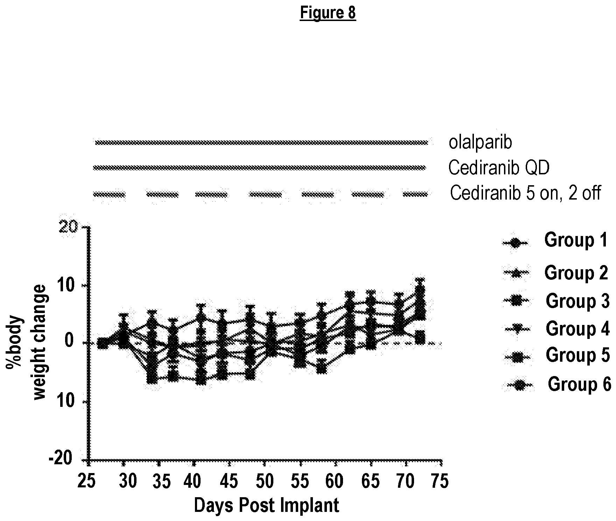

Figure 8 shows the body weight change for each group of tumour bearing animals in the study. This study compared cediranib and olaparib combinations where cediranib was administered once daily or once daily on a 5 days on 2 days off schedule. Group 1 - control, Group 2 - cediranib 3mg/kg once daily, Group 3 - cediranib 3mg/kg once daily 5 days on 2 days off, Group 4 - olaparib 100mg/kg once daily, Group 5 - cediranib 3mg/kg once daily plus olaparib 100mg/kg once daily, Group 6 - cediranib 3mg/kg once daily 5 days on 2 days off plus olaparib 100mg/kg once daily.

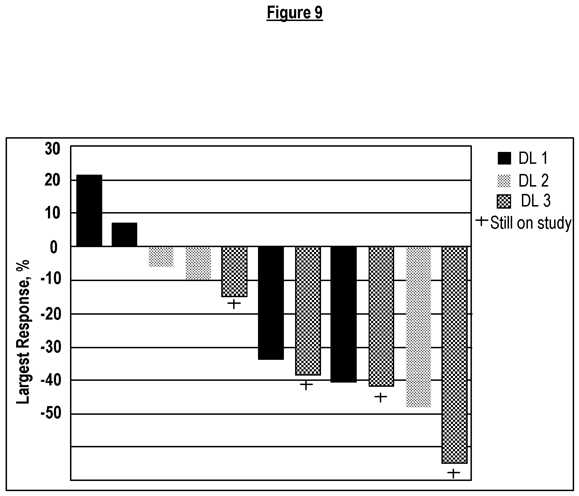

Figure 9 shows the largest response in the tumor size (from baseline) observed during the trial (i.e., the largest response observed may have been observed at any time point during the trial) for each patient in the different dosing regimen cohorts (DL1, DL2 and DL3 as described in Example 5). The black crosses indicate the patients receiving treatment according at data lock.

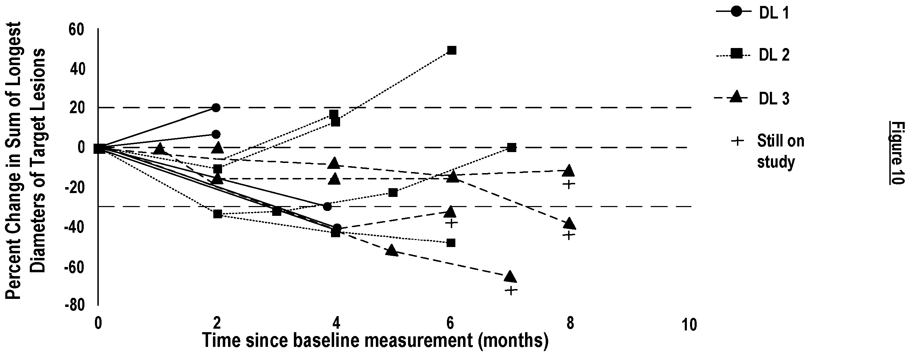

Figure 10 show the changes from baseline in the patient's tumor size over time for each patient in the different dosing regimen cohorts (DL1, DL2 and DL3 as described in Example 5).

As used herein, "treating" and "treatment" refer to the reduction or amelioration of the progression, severity and/or duration of a disease state, disorder, angiogenesis and/or vascular permeability effect or the amelioration of at least one symptom of any of the foregoing. In some embodiments, "treating" refers to an increase in progression-free survival.

In some aspects, the methods disclosed herein result in maintenance of tumor control. In some aspects, the methods disclosed herein allow administration of a reduced total dose of cediranib required to provide effective VEGF inhibition. In some aspects, the methods disclosed herein are methods of reducing adverse events and/or toxicity due to cediranib administration while maintaining cover on the VEGF pathway. In some aspects, the methods disclosed herein treat a warm blooded animal with platinum sensitive relapsed ovarian cancer. In some aspects, the methods disclosed herein improve the progression-free survival of patients. In some aspects, the improvement in the progression-free survival of patients is statistically significant. In some aspects, a statistically significant improvement in the progression-free survival of patients is when p < 0.05. In some aspects, a statistically significant improvement in the progression-free survival of patients is when p < 0.01.

In some aspects, the methods disclosed herein improve the overall survival of patients. In some aspects, the improvement in the overall survival of patients is statistically significant. In aspects, a statistically significant improvement in the overall survival of patients is when p < 0.05. In some aspects, a statistically significant improvement in the overall survival of patients is when p < 0.01.

Also disclosed is a composition comprising cediranib for use in producing an antiangiogenic and/or vascular permeability reducing effect in a warm-blooded animal, such as a human being, wherein the composition comprising cediranib is administered according to a fixed intermittent dosing regimen. In another aspect, there is disclosed a composition comprising cediranib for use in producing an antiangiogenic and/or vascular permeability reducing effect in a warm-blooded animal, such as a human being, wherein at least two cycles of the composition comprising cediranib is administered according to a fixed intermittent dosing regimen.

In another embodiment, there is disclosed herein a composition comprising cediranib for use in treating at least one disease state associated with angiogenesis in a human being, wherein at least two cycles of the composition comprising cediranib is administered according to a fixed dosing regimen, in accordance with the appended claims.

Also disclosed is a composition comprising cediranib for use in reducing the total dose of cediranib required to provide effective VEGF inhibition in a warm-blooded animal, such as a human being, wherein the composition comprising cediranib is administered according to a fixed dosing regimen. Also disclosed herein is a composition comprising cediranib for use in reducing the total dose of cediranib required to provide effective VEGF inhibition in a warm-blooded animal, such as a human being, wherein at least two cycles of the composition comprising cediranib is administered according to a fixed dosing regimen.

Also disclosed is a composition comprising cediranib for use in reducing adverse events and/or toxicity due to cediranib administration in a warm-blooded animal, such as a human being, wherein the composition comprising cediranib is administered according to a fixed dosing regimen. Also disclosed herein is a composition comprising cediranib for use in reducing adverse events and/or toxicity due to cediranib administration in a warm-blooded animal, such as a human being, wherein at least two cycles of the composition comprising cediranib is administered according to a fixed dosing regimen.

Also disclosed is a composition comprising cediranib for use in increasing repair of healthy, non-cancerous tissue during combination therapy treatment of cancer in a warm-blooded animal, such as a human being, wherein the composition comprising cediranib is administered according to a fixed dosing regimen. Also disclosed herein, is a composition comprising cediranib for use in increasing repair of healthy, non-cancerous tissue during combination therapy treatment of cancer in a warm-blooded animal, such as a human being, wherein at least two cycles of the composition comprising cediranib is administered according to a fixed dosing regimen.

Also disclosed is a composition comprising cediranib for use in treating a warm-blooded animal, such as a human being, with platinum sensitive relapsed cancer wherein the composition comprising cediranib is administered according to a fixed dosing regimen. Also disclosed herein is a composition comprising cediranib for use in treating a warm-blooded animal, such as a human being, with platinum sensitive relapsed cancer wherein at least two cycles of the composition comprising cediranib is administered according to a fixed dosing regimen.

Also disclosed is a composition comprising cediranib for use in the maintenance of tumor control. In another aspect, there is disclosed a composition comprising cediranib for use in reducing the total dose of cediranib required to provide effective VEGF inhibition. In another aspect, there is disclosed a composition comprising cediranib for use in reducing adverse events and/or toxicity due to cediranib administration while maintaining cover on the VEGF pathway. In another aspect, the compositions disclosed herein improve the progression-free survival of patients. In some aspects, a statistically significant improvement in the progression-free survival of patients is when p < 0.05. In some aspects, a statistically significant improvement in the progression-free survival of patients is when p < 0.01.

In another aspect, there is disclosed the use of a composition comprising cediranib for the manufacture of a medicament for producing an antiangiogenic and/or vascular permeability reducing effect in a warm-blooded animal, such as a human being, wherein the composition comprising cediranib is administered according to a fixed intermittent dosing regimen. In another aspect, there is disclosed the use of a composition comprising cediranib for the manufacture of a medicament for producing an antiangiogenic and/or vascular permeability reducing effect in a warm-blooded animal, such as a human being, wherein at least two cycles of the composition comprising cediranib is administered according to a fixed intermittent dosing regimen.

In another aspect, there is disclosed herein the use of a composition comprising cediranib for the manufacture of a medicament for treating at least one disease state associated with angiogenesis in a warm-blooded animal, such as a human being, wherein the composition comprising cediranib is administered according to a fixed dosing regimen. Also disclosed herein is the use of a composition comprising cediranib for the manufacture of a medicament for treating at least one disease state associated with angiogenesis in a warm-blooded animal, such as a human being, wherein at least two cycles of the composition comprising cediranib is administered according to a fixed dosing regimen.

In another aspect, there is disclosed the use of a composition comprising cediranib for the manufacture of a medicament for reducing the total dose of cediranib required to provide effective VEGF inhibition in a warm-blooded animal, such as a human being, wherein the composition comprising cediranib is administered according to a fixed dosing regimen. In one aspect, cover on the VEGF pathway is maintained. Also disclosed herein is the use of a composition comprising cediranib for the manufacture of a medicament for reducing the total dose of cediranib required to provide effective VEGF inhibition in a warm-blooded animal, such as a human being, wherein at least two cycles of the composition comprising cediranib is administered according to a fixed dosing regimen.

In another aspect, there is disclosed the use of a composition comprising cediranib for the manufacture of a medicament for reducing adverse events and/or toxicity due to cediranib administration in a warm-blooded animal, such as a human being, wherein the composition comprising cediranib is administered according to a fixed dosing regimen. Also disclosed herein is the use of a composition comprising cediranib for the manufacture of a medicament for reducing adverse events and/or toxicity due to cediranib administration in a warm-blooded animal, such as a human being, wherein at least two cycles of the composition comprising cediranib is administered according to a fixed dosing regimen.

In another aspect, there is disclosed the use of a composition comprising cediranib for the manufacture of a medicament for increasing repair of healthy, non-cancerous tissue during combination therapy treatment of cancer in a warm-blooded animal, such as a human being, wherein the composition comprising cediranib is administered according to a fixed dosing regimen. Also disclosed herein, is the use of a composition comprising cediranib for the manufacture of a medicament for increasing repair of healthy, non-cancerous tissue during combination therapy treatment of cancer in a warm-blooded animal, such as a human being, wherein at least two cycles of the composition comprising cediranib is administered according to a fixed dosing regimen.

In another aspect, there is disclosed the use of a composition comprising cediranib for the manufacture of a medicament for treating a warm-blooded animal, such as a human being, with platinum sensitive relapsed cancer wherein the composition comprising cediranib is administered according to a fixed dosing regimen. Also disclosed herein is the use of a composition comprising cediranib for the manufacture of a medicament for treating a warm-blooded animal, such as a human being, with platinum sensitive relapsed cancer wherein at least two cycles of the composition comprising cediranib is administered according to a fixed dosing regimen.

In another aspect, there is disclosed the use of a composition comprising cediranib for the manufacture of a medicament for maintenance of tumor control. In another aspect, there is disclosed the use of a composition comprising cediranib for the manufacture of a medicament for reducing the total dose of cediranib required to provide effective VEGF inhibition. In another aspect, there is disclosed the use of a composition comprising cediranib for the manufacture of a medicament for reducing adverse events and/or toxicity due to cediranib administration while maintaining cover on the VEGF pathway. In another aspect, the compositions disclosed herein improve the progression-free survival of patients. In some aspects, a statistically significant improvement in the progression-free survival of patients is when p < 0.05. In some aspects, a statistically significant improvement in the progression-free survival of patients is when p < 0.01.

As used herein, "effective amount" means an amount sufficient to elicit a desired biological response. As will be recognized by a person of ordinary skill in the art, the effective amount of cediranib may vary depending on various factors, such as the disease state being treated, the severity of disease state being treated, the desired effect of treatment, the warm-blooded animal in need of treatment, and the route of administration.

As used herein, "at least one disease state associated with angiogenesis" is chosen from lung cancers (e.g., non-small cell lung cancer (NSCLC) and small cell lung cancer (SCLC)); digestive and gastrointestinal cancers such as colorectal cancer, gastrointestinal stromal tumors, gastrointestinal carcinoid tumors, colon cancer, rectal cancer, anal cancer, bile duct cancer, small intestine cancer, and stomach (gastric) cancer; esophageal cancer; gallbladder cancer; liver cancer; pancreatic cancer; appendix cancer; breast cancer; ovarian cancer; renal cancer (e.g., renal cell carcinoma); cancers of the central nervous system; skin cancers; lymphomas; glioblastomas; choriocarcinomas; alveolar soft part sarcoma (ASPS); head and neck cancers (including thyroid cancers); osteogenic sarcomas; and blood cancers. Non-limiting examples of ovarian cancer include platinum sensitive ovarian cancers, platinum sensitive relapsed ovarian cancers, platinum insensitive ovarian cancers (resistant and refractory), high grade serous ovarian cancers, high grade endometrial ovarian cancer, clear cell ovarian cancers, mucinous ovarian cancers, and others. As used herein, ovarian cancer includes fallopian tube and peritoneal cancers, including primary peritoneal cancers.

As used herein, "an antiangiogenic and/or vascular permeability reducing effect" can be assessed by a variety of assays and tests known to one of ordinary skill in the art including, for example, those measuring inhibition of tyrosine kinase activity associated with VEGF receptors such as Flt and/or KDR. These properties may be assessed, for example, by one or more of: (a) In Vitro Receptor Tyrosine Kinase Inhibition Test, (b) In Vitro HUVEC Proliferation Assay, (c) In Vivo Solid Tumor Disease Model. Non-limiting examples of such assays are set out below but one of ordinary skill will recognize that other procedures are equally suitable.

As used herein, the term "fixed intermittent dosing regimen" refers to repeating cycles of preplanned drug administration in which the drug is administered on one or more consecutive days ("days on") followed by one or more consecutive days of rest on which the drug is not administered ("days off').

The cycles are regular, in that the pattern of days on and days off is the same in each cycle. In some embodiments, each of the repeating cycles, however, is preplanned in that it is not determined solely in response to the appearance of one or more adverse events.

In some embodiments, administration of the composition comprising cediranib is repeated for two to ten cycles, such as for example two cycles, three cycles, four cycles, five cycles, six cycles, seven cycles, eight cycles, nine cycles or ten cycles.

A cycle consists of 7 days.

The fixed intermittent dosing regimen comprises a repeating cycle of administration of an effective amount of said composition comprising cediranib on 5 consecutive days followed by 2 days of rest.

In some embodiments, placebo is administered on said days of rest.

In some embodiments, the fixed intermittent dosing regimen comprises administering cediranib orally at a dose of 20 mg once daily for five days of a seven day cycle followed by two days of placebo doses. In some embodiments, the fixed intermittent dosing regimen comprises administering cediranib orally at a dose of 30 mg once daily for five days of a seven day cycle followed by two days of placebo doses.

Compositions comprising cediranib suitable for use in the present invention may be in a form suitable for oral administration (for example, as tablets, lozenges, hard or soft capsules, aqueous or oily suspensions, emulsions, dispersible powders or granules, syrups or elixirs), for administration by inhalation (for example, as a finely divided powder or a liquid aerosol), for administration by insufflation (for example, as a finely divided powder), for parenteral injection (for example, as a sterile solution, suspension or emulsion for intravenous, subcutaneous, intramuscular, intravascular or infusion dosing), for topical administration (for example as creams, ointments, gels, or aqueous or oily solutions or suspensions), or for rectal administration (for example as a suppository). In some embodiments, compositions comprising cediranib are administered orally.

In some embodiments, compositions comprising cediranib may be prepared in a conventional manner using at least one conventional excipient. In some embodiments, compositions comprising cediranib consist of cediranib. In some embodiments, compositions comprising cediranib further comprise at least one pharmaceutically acceptable excipient or carrier. In some embodiments, compositions comprising cediranib comprise cediranib as the sole pharmaceutically active ingredient. In some embodiments, compositions comprising cediranib further comprise at least one additional pharmaceutically active ingredient.

Doses of cediranib may vary according to therapeutic requirements. In some embodiments, the compositions comprising cediranib are in unit dosage form. In some embodiments, the compositions comprising cediranib are administered to a warm-blooded animal at a unit dose within a range of 1-50 mg per square meter body area of the animal, for example approximately 0.03 mg/kg to 1.5 mg/kg in a human. In some embodiments, unit doses range, for example, from 0.01 mg/kg to 1.5 mg/kg, further for example from 0.05 mg/kg to 0.75 mg/kg, and further for example from 0.03 mg/kg to 0.5 mg/kg.

In some embodiments, the compositions comprising cediranib are administered at dose of 0.6 mg/kg. In some embodiments, the compositions comprising cediranib are administered at dose of 1.2 mg/kg. In some embodiments, the compositions comprising cediranib are administered at dose of 2.4 mg/kg. In some embodiments, the compositions comprising cediranib are administered at dose of 4.8 mg/kg.

In some embodiments, a solid dosage form comprises cediranib in an amount ranging from 0.5 mg to 90 mg as measured by weight of the free base of cediranib. In some embodiments, a solid dosage form comprises cediranib in an amount ranging from 1 mg to 50 mg as measured by weight of the free base of cediranib. In some embodiments, the solid dosage form comprises cediranib in an amount ranging from 5 mg to 50 mg, such as from 10 mg to 40 mg, a such as from 15 mg to 35 mg, and further such as from 20 mg to 30 mg as measured by weight of the free base of cediranib. In some embodiments, the solid dosage form comprises cediranib in an amount of 30 mg as measured by weight of the free base of cediranib. In some embodiments, the solid dosage form comprises cediranib in an amount of 20 mg as measured by weight of the free base of cediranib. In some embodiments, the solid dosage form comprises cediranib in an amount of 15 mg as measured by weight of the free base of cediranib.

In some embodiments, the daily dose of cediranib ranges from 5 mg to 50 mg, such as from 15 mg to 35 mg, and further such as from 20 mg to 30 mg as measured by weight of the free base of cediranib. In some embodiments, the daily dose of cediranib is 30 mg as measured by weight of the free base of cediranib. In some embodiments, the daily dose of cediranib is 20 mg as measured by weight of the free base of cediranib. In some embodiments, the daily dose of cediranib is 15 mg as measured by weight of the free base of cediranib. In some embodiments, the daily dose of cediranib is 10 mg as measured by weight of the free base of cediranib.

In some embodiments, cediranib is administered in a solid dosage form, such as a tablet, comprising the weight equivalent of 30 mg of the free base of cediranib, which is 37.8 mg of cediranib, the maleate salt. In some embodiments, cediranib is administered in a solid dosage form, such as a tablet, comprising the weight equivalent of 20 mg of the free base of cediranib, which is 25.2 mg of cediranib, the maleate salt. In some embodiments, cediranib is administered in a solid dosage form comprising the weight equivalent of 15 mg of the free base of cediranib, which is 18.9 mg of cediranib, the maleate salt.

In some embodiments, cediranib is administered once daily. In some embodiments, cediranib is administered twice daily. In some embodiments, cediranib is administered three times daily. In some embodiments, cediranib is administered four times daily. In some embodiments, cediranib is administered five times daily.

In some embodiments, cediranib is administered to a warm-blooded animal, such as a human being, with an empty stomach, such as, for example, at least one 1 hour before or at least 2 hours after a meal.

In some embodiments, such as where the warm-blooded animal has difficulty swallowing tablets, cediranib tablets are dispersed in non- carbonated drinking water. In some embodiments, a cediranib dispersion is administered through nasogastric or gastrostomy tubes.

As disclosed above, cediranib may be used as a monotherapy or as part of a combination therapy that may involve, in addition to cediranib, at least one other component chosen from partner drugs and other treatments. Such combination therapy may be achieved by way of simultaneous, sequential, and/or separate administration of the individual components (cediranib compositions and at least one other component) of the treatment. In some embodiments, the individual components are administered simultaneously. In some embodiments, the individual components are administered separately. In some embodiments, the individual components are administered sequentially.

Angiogenesis and VEGR2-mediated maintenance of vascular function as well as control of hypertension is important for many normal tissues, and long term suppression of VEGFR signaling can lead to stress in normal tissues that can be manifested as clinical observations such as fatigue, modification in thyroid function, and diarrhea. When combined with at least one other component that also can have significant impact on normal tissues, for example in particular those that can cause damage that requires repair, then continuous dosing of cediranib may delay and/or prevent repair of normal tissues. Accordingly, administering cediranib using the fixed intermittent dosing regimen disclosed herein with regular, short intermittent breaks (either as monotherapy or combination) in a schedule may allow repair of normal tissue but without allowing the tumor to recover. Moreover, the short breaks may ensure that there is no issue with co-medications such as, for example, anti-hypertensives.

In the field of medical oncology, it is common to use a combination of different forms of treatment to treat patients with cancer. In medical oncology, the at least one other component(s) of such combination therapy treatment in addition to administration of cediranib may be chosen from surgery, radiotherapy, and chemotherapy. Such chemotherapy may include at least one partner drug. For partner drugs that have greater impact on normal tissues, such as for example chemotherapies, the same strategy applies. Around times when the damage is greatest then slightly longer preplanned dose interruptions may be appropriate, for example, interruption of 4-5 days. Non-limiting examples of partner drugs include:
DNA damage response inhibitors (such as, for example, PARP inhibitors (such as, for example, olaparib (Lynparza)), Wee-1 inhibitors, ATR inhibitors, ATM inhibitors, and DNAPK inhibitors),
immune checkpoint modulators (such as, for example, anti-PD-1 antibodies, anti-PD-Ll antibodies (such as, for example, MEDI4736 (durvalumab)), anti-CTLA4 antibodies, TLR7 agonists, CD40 agonists, Lag-3 antagonists, and OX40 agonists),
tumor cell targeting therapy agents (such as, for example, EGFR, Her2, MAPK/raf, Met, Pi3K, mTOR, Akt, estrogen antagonists, androgen targeted therapeutics, FGFR, MCT-1 and MCT-4 inhibitors), and
chemotherapy agents (such as for example, platinum based chemotherapy, taxane based chemotherapy, and irotecan), including:
   (i) other antiangiogenic agents such as alkylating agents (for example, cisplatin, oxaliplatin, carboplatin, cyclophosphamide, nitrogen mustard, melphalan, chlorambucil, busulphan, bendamustine, temozolamide, nitrosoureas, and thiotepa); antimetabolites (for example, gemcitabine and antifolates such as fluoropyrimidines like 5-fluorouracil and tegafur, raltitrexed, methotrexate, cytosine arabinoside, hydroxyurea and purine analogues such as fludarabine, and adenosine analogues); antitumor antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine, taxoids like taxol and taxotere, and polokinase inhibitors); and topoisomerase inhibitors (for example epipodophyllotoxins like etoposide and teniposide, amsacrine, topotecan, camptothecin, and irinotecan); enzymes (for example asparaginase); and thymidylate synthase inhibitors (for example raltitrexed);
   (ii) cytostatic agents such as antioestrogens (for example tamoxifen, toremifene, raloxifene, droloxifene, and iodoxyfene,), androgen receptor down regulators (for example fulvestrant), antagonists MDV3100 or ARN-509 which prevent nuclear translocation of the androgen receptor and its binding to either DNA or coactivator proteins, inhibitors of CYP17A1 such as abiraterone [ZYTIGA^{™}], and mixed inhibitors of androgen receptor function and CYP17A1 such as TOK-001 (galeterone), LHRH antagonists and LHRH agonists (for example goserelin, goserelin acetate, luprolide, leuprorelin and buserelin), progestogens (for example megestrol acetate), aromatase inhibitors (for example anastrozole, letrozole, vorazole, and exemestane), antiprogestogens, antiandrogens (for example flutamide, nilutamide, bicalutamide, and cyproterone acetate), and inhibitors of 5α-reductase (for example finasteride),
   (iii) anti-invasion agents (for example c-Src kinase family inhibitors like 4-(6-chloro-2,3-methylenedioxyanilino)-7-[2-(4-methylpiperazin-1-yl)ethoxy]-5-tetrahydropyran-4-yloxyquinazoline (AZD0530; International Patent Application WO 01/94341), N-(2-chloro-6-methylphenyl)-2-{6-[4-(2-hydroxyethyl)piperazin-1-yl]-2-methylpyrimidin-4-ylamino}thiazole-5-carboxamide (dasatinib, BMS-354825; J. Med. Chem., 2004, 47, 6658-6661) and bosutinib (SKI-606), metalloproteinase inhibitors like marimastat, inhibitors of urokinase plasminogen activator receptor function, and antibodies to Heparanase),
   (iv) inhibitors of growth factor function, such as inhibitors of platelet derived growth factor and inhibitors of hepatocyte growth factor, such as growth factor antibodies and growth factor receptor antibodies (for example the anti erbB2 antibody trastuzumab [Herceptin^{™}], the anti-EGFR antibody panitumumab, the anti erbB1 antibody cetuximab [Erbitux, C225]) and inhibitors of any other growth factor antibodies or other growth factor receptor antibodies, such as farnesyl transferase inhibitors (for example those disclosed by Stern et al. Critical reviews in oncology/haematology, 2005, Vol. 54, p. 11-29); such inhibitors also include tyrosine kinase inhibitors for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)) and serine/threonine kinase inhibitors), erbB2 tyrosine kinase inhibitors (such as lapatinib); inhibitors of the hepatocyte growth factor family; inhibitors of the insulin growth factor family; inhibitors of the plateletderived growth factor family such as imatinib and/or nilotinib (AMN107); inhibitors of serine/threonine kinase inhibitors kinases (for example Ras/Raf signalling inhibitors such as farnesyl transferase inhibitors, for example sorafenib (BAY 43-9006), tipifarnib (R115777) and AZD9291 (tagrisso); lonafarnib (SCH66336)), inhibitors of cell signalling through MEK and /or AKT kinases, c-kit inhibitors, abl kinase inhibitors, PI3 kinase inhibitors, Plt3 kinase inhibitors, CSF-1R kinase inhibitors, IGF receptor (insulin-like growth factor) kinase inhibitors; aurora kinase inhibitors (for example AZD1152, PH739358, VX-680, MLN8054, R763, MP235, MP529, VX-528 AND AX39459) and cyclin dependent kinase inhibitors such as CDK2 and/or CDK4 inhibitors;
   (v) other antiangiogenic agents such as those that work by different mechanisms from those defined hereinbefore (for example Ang-2 (such as MEDI-3617) and DLL4 (such as MEDI-0639)), and including vascular targeting agents (for example combretastatin phosphate (Combretastatin A4) and compounds disclosed in International Patent Application Publication Nos. WO 99/02166, WO 00/40529, WO 00/41669, WO 01/92224, WO 02/04434 and WO 02/08213 and the vascular damaging agents described in International Patent Application Publication No. WO 99/02166 (for example N-acetylcolchinol-O-phosphate)); and
   (vi) endothelin receptor antagonists (for example zibotentan (ZD4054) and atrasentan);
biological response modifiers (for example, interferon);
antibodies (for example edrecolomab);
antisense therapies (for example those which are directed to the targets listed above, such as ISIS 2503, an anti-ras antisense);
gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
immunotherapy approaches, including for example ex-vivo and in vivo approaches to increase the immunogenicity of patient tumor cells, such as transfection with cytokines such as interleukin 2, interleukin 4 or granulocyte-macrophage colony stimulating factor, approaches to decrease T-cell energy, approaches using transfected immune cells such as cytokine-transfected dendritic cells, approaches using cytokine-transfected tumor cell lines and approaches using anti-idiotypic antibodies, approaches for T-cell enhancement including CTLA4 antibodies, and antibodies directed toward CD137, PD-1 or B7-H1, tollreceptor agonists; agonistic antibodies to CD40 such as SGN-40 (Dacetuzumab) or to the Tweak receptor such as PDL-192; agonistic antibodies to FAS; approaches using antibodies to tumor associated antigens, and antibodies that deplete target cell types (e.g. unconjugated anti-CD20 antibodies such as Rituximab, ofatumumab, Obinutuzumab, anti-CD 19 antibodies such as MEDI-551, anti-CD52 antibodies such as Alemtuzumab, anti-CD37 antibodies such as TRU-016, anti-CD22 antibodies such as Inotuzumab, radiolabeled anti-CD20 antibodies Bexxar and Zevalin, and anti-CD54 antibody Campath; immunotoxins such as moxetumumab pasudotox), approaches using anti-idiotypic antibodies, approaches that enhance Natural Killer cell function, and approaches that utilize antibody-toxin conjugates (e.g. anti-CD33 antibody Mylotarg), and immune modifiers such as Revlimid (Lenalidomide).

As used herein, "anti-PD-Ll antibody" means an antibody that selectively binds a PD-L1 polypeptide. Exemplary anti-PD-Ll antibodies are described for example at US 8,779,108 and US 9,493,565. MEDI4736 is an exemplary anti-PD-Ll antibody. Other anti-PD-Ll antibodies include BMS-936559 (Bristol-Myers Squibb) and MPDL3280A (Roche).
**MEDI4736 VL** (SEQ ID NO: 1)
**MEDI4736 VH** (SEQ ID NO: 2)
**MEDI4736 VH CDR1** (SEQ ID NO: 3)
   RYWMS
**MEDI4736 VH CDR2** (SEQ ID NO: 4)
   NIKQDGSEKYYVDSVKG
**MEDI4736 VH CDR3** (SEQ ID NO: 5)
   EGGWFGELAFDY
**MEDI4736 VL CDR1** (SEQ ID NO: 6)
   RASQRVSSSYLA
**MEDI4736 VL CDR2** (SEQ ID NO: 7)
   DASSRAT
**MEDI4736 VL CDR3** (SEQ ID NO: 8)
   QQYGSLPWT

The at least one partner drug may be administered at the recommended dose(s) and according to the recommended dose regimen(s). For example, MEDI4736 (durvalumab) may be administered at a dose of 3 mg/kg or 10 mg/kg IV every 2 weeks or as a fixed dose of 1500 mg every 4 weeks. Further for example, olaparib (Lynparza) may be administered orally in the form of a 150 mg or 200 mg or 300 mg tablet BID.

In some embodiments, the human being has relapsed ovarian cancers, fallopian tube or primary peritoneal cancers. In some embodiments, to said human being is administered cediranib according to the fixed intermittent dosing regimen of claim 1 in combination with at least one partner drug chosen from (a) platinum-based chemotherapy, (b) olaparib (Lynparza) and (c) durvalumab, each optionally followed by maintenance cediranib monotherapy in accordance with the fixed intermittent dosing regimen of claim 1. In some embodiments, to said human being is administered cediranib according to the fixed intermittent dosing regimen of claim 1 in combination with at least two partner drug chosen from (a) platinum-based chemotherapy, (b) olaparib (Lynparza) and (c) durvalumab, each optionally followed by maintenance cediranib monotherapy in accordance with the fixed intermittent dosing regimen of claim 1. In some embodiments, to said human being is administered cediranib according to the fixed intermittent dosing regimen of claim 1 in combination with platinum-based chemotherapy, olaparib (Lynparza) and durvalumab, each optionally followed by maintenance cediranib monotherapy in accordance with the fixed intermittent dosing regimen of claim 1. In some embodiments, to said human being is administered cediranib according to the fixed intermittent dosing regimen of claim 1 in combination with olaparib (Lynparza) and durvalumab, each optionally followed by maintenance cediranib monotherapy in accordance with the fixed intermittent dosing regimen of claim 1.

In some embodiments, cediranib is administered according to the fixed intermittent dosing regimen according to claim 1 in combination with platinum-based chemotherapy, and followed by maintenance monotherapy in accordance with the fixed intermittent dosing regimen of claim 1, for the treatment of adult patients with platinum sensitive relapsed (PSR) ovarian cancer (including fallopian tube, high grade endometrial, clear cell, high grade serous or primary peritoneal). In some embodiments, cediranib is administered according to the fixed intermittent dosing regimen of claim 1 in combination with platinum-based chemotherapy, and followed by maintenance monotherapy in accordance with the fixed intermittent dosing regimen of claim 1, for the treatment of adult patients with platinum sensitive relapsed (PSR) ovarian cancer (including fallopian tube or primary peritoneal). A composition comprising cediranib is provided for use in method of treating a warm blooded animal with platinum sensitive relapsed ovarian cancer, wherein said method comprises at least two cycles of administration of a composition comprising cediranib according to a fixed intermittent dosing regimen, said fixed intermittent dosing regimen comprising administration of an effective amount of said composition on at least two consecutive days of a cycle followed by at least two consecutive days of a cycle on which said composition is not administered, and further comprising administering platinum-based chemotherapy. Platinum sensitive relapsed ovarian cancer may be chosen from fallopian tube cancers, high grade endometrial, clear cell, high grade serous and primary peritoneal cancers. Platinum sensitive relapsed ovarian cancer may be chosen from fallopian tube and primary peritoneal cancers. The method may further comprise maintenance monotherapy of cediranib. Maintenance monotherapy of cediranib comprises administration of a composition comprising cediranib according to the fixed intermittent dosing regimen according to claim 1.

In some embodiments, cediranib is administered according to a continuous dosing regimen, and followed by cediranib being administered according to a fixed intermittent dosing regimen. The continuous dosing regimens comprise administration of an effective amount of a composition comprising cediranib on one or more consecutive days. The fixed intermittent dosing regimens comprise administration of an effective amount of a composition comprising cediranib on two or more consecutive days of a cycle, followed by two consecutive days of rest, on which said composition is not administered. These comprise only the use of compositions comprising cediranib and thus may be used in monotherapy or may further comprise the administration of one or more partner drugs and thus may be used in combination therapy.

Clinical trials using the compositions disclosed herein are currently being planned.

For example, one planned clinical trial is a randomized, double-blind, parallelgroup, international study to evaluate the safety, tolerability and efficacy of 2 regimens of cediranib in combination with platinum-based chemotherapy in patients with platinum sensitive relapsed epithelial ovarian cancer, primary peritoneal and/or fallopian tube cancer. The proposed protocol involves randomization of subjects to receive 1 of the 2 following treatment regimens during 2 phases, a combination chemotherapy phase (up to 6 cycles) and a maintenance phase (until progression): (1) cediranib 20 mg orally once daily ("continuous" regimen) or (2) cediranib 20 mg orally once daily to be administered on a fixed intermittent regimen for five consecutive days of a seven day cycle followed by 2 consecutive days of placebo doses. All subjects will also concurrently receive 6 cycles of platinum-based chemotherapy. Subjects will be treated during the combination chemotherapy phase with a carboplatin regimen. Non-limiting examples of the carboplatin regimen include:
- carboplatin area under the concentration time curve 5 (AUC 5; glomerular filtration rate [GFR] measured) over 30 to 60 minutes, in combination with paclitaxel 175 mg/m² over 3 hours, once every 3 weeks (1 cycle) for 6 cycles (q3w × 6),
- carboplatin AUC 5 (GFR measured) over 30 to 60 minutes, in combination with PLD 30 mg/m² over 3 hours, once every 4 weeks (1 cycle) for 6 cycles (q4w × 6), and
- carboplatin AUC 4 (GFR) over 30 to 60 minutes (on Day 1) in combination with gemcitabine 1000 mg/m2 (on Days 1 and 8) over 3 hours, once every 3 weeks (1 cycle) for 6 cycles.

Safety and tolerability of a fixed intermittent cediranib regimen as compared to a continuous regimen will be measured by cediranib discontinuation rate due to toxicity defined by any adverse event or subject decision leading to discontinuation of cediranib within 6 months of randomization. The primary endpoint for this study is the proportion of subjects who discontinue cediranib treatment due to adverse event or subject's decision within 6 months of randomization. Analysis will be carried out once all subjects reach 6 months of treatment or discontinue, using the full analysis set consisting of all randomized subjects. All subjects will have Response Evaluation Criteria in Solid Tumors (RECIST Version 1.1) tumor assessments at screening (within 28 days of randomization) and every 12 weeks (±1 week) after randomization until objective radiological disease progression.

In some embodiments, the fixed intermittent regimen decreases the rate of discontinuation due to toxicity compared to continuous regimen. Efficacy of a fixed intermittent cediranib regimen as compared to a continuous regimen may be measured using one or more of the following endpoints: progression-free survival, overall survival, time to treatment failure, and objective response rate.

Progression-free survival (PFS) is defined as time from randomization to first documentation of objective disease progression as determined by independent radiology review or to death on study due to any cause, whichever occurs first.

Overall survival (OS) is defined as the time from randomization to the date of death due to any cause. For subjects still alive at the time of analysis, the OS time will be censored on the last date the subjects were known to be alive.

Time to treatment failure (TTF) is defined as time from randomisation to treatment failure.

Objective response rate (ORR) is defined as the percentage of subjects with a complete response (CR) or partial response (PR) according to RECIST (as determined by independent radiology review), relative to the total population of all randomized subjects.

Another planned clinical trial is a Phase I/II study of the anti-programmed death ligand-1 antibody MEDI4736 (durvalumab) in combination with cediranib for advanced solid tumors and advanced or recurrent ovarian, triple negative breast, lung, prostate and colorectal cancers. Dose schedules are shown in Table 1 and Table 2.

**Table 1: Durvalumab + Cediranib Daily Schedule Dose Escalation Table**

| **Dose Level (DL)** | **Durvalumab (intravenously, for 12mo)** | **Cediranib (oral, once daily, continuous)** |
|---|---|---|
| DL-2 | 3 mg/kg every 2 weeks | 15 mg |
| DL-1 | 3 mg/kg every 2 weeks | 20 mg |
| **DL 1 (starting dose)** | **10 mg/kg every 2 weeks** | **20 mg** |
| DL 2 | 10 mg/kg every 2 weeks | 30 mg |

**Table 2: Durvalumab + Cediranib Intermittent Schedule Dose Escalation Table**

| **Dose Level (DL)** | **Durvalumab (intravenously, for 12mo)** | **Cediranib (oral, 5 days on/2 days off)** |
|---|---|---|
| DL-2 | A fixed dose of 500 mg every 4 weeks | 15 mg |
| DL-1 | A fixed dose of 1500 mg every 4 weeks | 15 mg |
| **DL 1 (starting dose)** | **A fixed dose of 1500 mg every 4 weeks** | **20 mg** |

For the durvalumab + cediranib arm, eligible patients will have been diagnosed with advanced or recurrent Ovarian Cancer (Cohort 1), NSCLC (Cohort 2), or CRC (Cohort 3). In Phase I, which used a continuous dosing regimen for administration of cediranib, 2 patients on DL1 required early discontinuation of cediranib due to pulmonary thromboembolism and 1 patient on DL1 had dose reduction to cediranib 15 mg daily due to recurrent grade 2 fatigue on cycle 2. Three of 4 patients on DL2 also had cediranib dose reduction to 20 mg daily due to recurrent grade 2 fatigue, grade 2 abdominal pain and grade 2 diarrhea during cycles 2-3.

The inventor of the present disclosure discovered that preclinical *in vivo* data showed no difference in anti-tumor activity with intermittent cediranib schedules (5 days on/2 days off) compared to a daily cediranib schedule. Thus, new durvalumab + cediranib dose levels with an intermittent cediranib schedule (5 days on/2 days off) according to the present disclosure with durvalumab at a fixed dose of 1500 mg every 28 days will be used in the clinical trials to determine whether there is, for example, an improvement in tolerability. Specifically, an intermittent cediranib dose schedule according to the present disclosure was investigated and a Phase II study of durvalumab +cediranib in ovarian cancer, NSCLC, and CRC is currently opened.

### EXAMPLES

Examples falling outside the scope of the appended claims are provided for reference purposes only.

**Example 1: In Vivo Solid Tumor Disease Model Assay.** This assay can measure the capacity of compounds to inhibit solid tumor growth.

The following is an example of a typical procedure that may be used.

CaLu-6 tumor xenografts may be established in the flank of female athymic Swiss nulnu mice, by subcutaneous injection of 1×10 CaLu-6 cells/mouse in 100pu of a 50% (v/v) solution of Matrigel in serum free culture medium. Ten days after cellular implant, mice may be allocated to groups of 8-10, so as to achieve comparable group mean volumes. Tumors may be measured using vernier calipers and volumes may be calculated as: (I × w) x i (l × w) × (z/6), where I is the longest diameter and w the diameter perpendicular to the longest. Test compounds may be administered orally once daily for a minimum of 21 days, and control animals received compound diluent. Tumors may be measured twice weekly. The level of growth inhibition may be calculated by comparison of the mean tumor volume of the control group versus the treatment group using a Student T test and/or a Mann-Whitney Rank Sum Test. The inhibitory effect of compound treatment may be considered significant when p < 0.05.

**Example 2: Assessing target cover.** To establish the target cover achieved with cediranib in pre-clinical models, the time dependent PK profile was determined at a range of compound doses. The mean free drug profile of cediranib modelled based on multiple dosing at 0.6, 1.2, 2.4 and 4.8 mg/kg was calculated and aligned against the ICso for potency versus VEGFR-1, VEGFR-2, c-kit and PDGFR.

Figures 1A-P show modelled mean free plasma concentrations of cediranib over time in pre-clinical models dosed at a). 0.6 mg/kg (Figures 1A-1D), b). 1.2 mg/kg (Figures 1E-1H), c). 2.4 mg/kg (Figures 1I-1L), or d). 4.8 mg/kg (Figures 1M-1P) following dosing regimens of once daily continuous (QDCont), once daily 5 days on 2 days off (QD5on2off), or once daily 4 days (QD4on3off). In addition, the PK of cediranib following a redose after a 2 or 3 day break is shown. The reduction in PK is shown following the last dose of drug (at either 4 or 5 days dosing), and overlaid on each plot (horizontal lines) are the cellular ICso values generated *in vitro* for inhibition of pVEGFR-1,-2, pKit, pPDGFRα and pPDGFRβ, as indicated. For the plots exemplifying VEGFR and Kit cover, R-P stands for receptor phosphorylation in a cell based assay; C-P stands for cell proliferation; for VEGFR plots, T-G stands for tubule growth in an endothelial-fibroblast co-culture assay. The relevant cell lines are indicated. For the graphs depicting PDGFRα and β cover, inhibition of PDGFRα and PDGFRβ phosphorylation and PDGFBB or PDGFAA driven proliferation are shown together with the relevant cell lines. The plots in Figure 1A-P exemplify the preclinical PK profile for cediranib at a range of doses and show that target cover can be lost rapidly following the last dose. This also established that at doses up to 2.4 mg/kg cediranib was achieving target cover versus VEGFRs and c-kit but was not giving sufficient cover versus PDGFR. It also demonstrated that, following the final dose in a given treatment cycle, the dose interruption can relieve suppression of the VEGFR signaling.

To establish the target cover achieved with cediranib in humans, the mean time dependent free drug PK profile from a population PK analysis was plotted, and similarly aligned versus the same IC₅₀ values versus VEGFR-1,VEGFR-2, c-kit and PDGFRs. This analysis demonstrated that the 20 mg and 30 mg has a similar target cover to that achieved in the 1.2-2.4 mg/kg range pre-clinically. It also showed that, when drug dosing is interrupted, cover versus VEGFR and c-kit are lost with hours and that a 2 day (or more) break can relieve suppression of the VEGFR signaling.

Figure 2 shows modelled mean the free drug exposure in humans for cediranib (black line) versus time for 20 mg and 15 mg doses of cediranib. The 95% confidence intervals are represented by the grey ribbon. The PK curve shows the reduction in cediranib concentration following the final dose of compound. Overlaid on the plot (horizontal lines) are the cellular IC₅₀ values generated in vitro for inhibition of pVEGFR, pKit, pPDGFRs. This data confirms that the free drug levels observed in the clinic are in the range of exposures observed when cediranib is dose at 1.2 - 2.4 mg/kg preclinically, and that the PK profile is similar to that observed pre-clinically.

**Example 3: To assess maintenance of anti-tumor benefit.** To determine whether intermittent dosing of cediranib is able to maintain anti-tumor benefit, a range of tumor xenograft models implanted sub-cutaneously into nude or scid mice representing differential sensitivity to cediranib were used. Tumors were selected and randomized into groups when tumor volume reached approximately 0.2 cm³. SW620 (CRC model), Calu6 (NSCLC model) and A498 (renal cancer model) tumors were dosed once daily orally with 1.2 mg/kg and 2.4 mg/kg (equivalent to 1.5 and 3 mg/kg cediranib maleate salt). To test the impact of the intermittent dosing, cediranib was dosed once daily orally for 7 days, 5 days or 4 days out of each 7 days cycle. Groups of 10 animals were used, however in the final cycle cohorts of 5 animals were removed in between dosing group to support assessment of pharmacodynamic biomarkers in the tumor. This data demonstrated that the 5 days on 2 days off schedule maintains anti-tumor effects as monotherapy despite relief of VEGFR suppression. Moreover the 4 days on 3 days off schedule also maintains antitumor effects albeit at not as effectively as the 5/2 schedule. This data established that a specific intermittent dosing strategy can maintain anti-tumor effects of cediranib. This presents the opportunity to give short structured breaks in cediranib treatment as either a monotherapy or a combination therapy with drugs targeting other mechanism (e.g., DNA damaging agents, immunotherapy, or tumor cell targeted therapy). (Figures 3-6).

Figure 3 shows mean tumor volume versus time from three efficacy studies (performed in Calu6, A498, and SW620 tumor xenografts) each using four oral dosing regimen groups (Group 1 (Vehicle once daily for 21 days), Group 2 (1.5 mg/kg cediranib once daily for 21 days), Group 3 (3 mg/kg cediranib once daily for 21 days), and Group 6 (three 7-day cycles of 1.5 mg/kg cediranib once daily for 5 days followed by vehicle once daily for 2 days. This data shows that intermittent dosing of cediranib at 1.5 mg/kg on a 5/2 schedule maintains efficacy.

Figure 4 shows mean tumor volume versus time from three efficacy studies (in Calu6, A498, and SW620 tumor xenografts) each using four oral dosing regimen groups (Group 1 (Vehicle once daily for 21 days), Group 2 (1.5 mg/kg cediranib once daily for 21 days), Group 3 (3 mg/kg cediranib once daily for 21 days), and Group 7 (three 7-day cycles of 3 mg/kg cediranib once daily for 5 days followed by vehicle once daily for 2 days. This data shows that intermittent dosing of cediranib at 3 mg/kg on a 5/2 schedule maintains efficacy.

Figure 5 shows mean tumor volume versus time from three efficacy studies (Calu6, A498, and SW620) each using four oral dosing regimen groups (Group 1 (Vehicle once daily for 21 days), Group 2 (1.5 mg/kg cediranib once daily for 21 days), Group 3 (3 mg/kg cediranib once daily for 21 days), and Group 8 (three 7-day cycles of 1.5 mg/kg cediranib once daily for 3 days followed by vehicle once daily for 4 days. This data shows that in 2 out of the three models intermittent dosing of cediranib at 1.5 mg/kg on a 4/3 schedule maintains efficacy.

Figure 6 shows mean tumor volume versus time from three efficacy studies (Calu6, A498, and SW620) each using four oral dosing regimen groups (Group 1 (Vehicle once daily for 21 days), Group 2 (1.5 mg/kg cediranib once daily for 21 days), Group 3 (3 mg/kg cediranib once daily for 21 days), and Group 9 (three 7-day cycles of 3 mg/kg cediranib once daily for 3 days followed by vehicle once daily for 4 days)). This data shows that intermittent dosing of cediranib at 3 mg/kg on a 4/3 schedule maintains efficacy.

### Example 4: To test the benefit of cediranib in combination with olaparib using an intermittent schedule, an intermittent (5 days on 2 days off) daily dose of cediranib combined with olaparib is as efficacious as a constant daily dose of cediranib combined with olaparib.

To establish that giving an intermittent dose of cediranib does not reduce antitumour efficacy of cediranib alone or in combination with olaparib, OV2022 tumour (ovarian cancer) xenografts were treated with 3 mg/kg cediranib daily or on a 5 days on, 2 days off schedule alone or in combination with 100 mg/kg olaparib. The intermittent schedule of cediranib gave equivalent efficacy to the constant dose of cediranib, thus showing that the intermittent dose of cediranib may be used effectively.

Figure 7 shows mean tumour volume versus time from an efficacy study performed in the OV2022 (ovarian cancer) patient derived tumour xenograft model. This study compared cediranib and olaparib combinations where cediranib was administered once daily or once daily on a 5 days on 2 days off schedule. Group 1 - control, Group 2 - cediranib 3mg/kg once daily, Group 3 - cediranib 3mg/kg once daily 5 days on 2 days off, Group 4 - olaparib 100mg/kg once daily, Group 5 - cediranib 3mg/kg once daily plus olaparib 100mg/kg once daily, Group 6 - cediranib 3mg/kg once daily 5 days on 2 days off plus olaparib 100mg/kg once daily.

Figure 8 shows the body weight change for each group of tumour bearing animals in the study. This study compared cediranib and olaparib combinations where cediranib was administered once daily or once daily on a 5 days on 2 days off schedule. Group 1 - control, Group 2 - cediranib 3mg/kg once daily, Group 3 - cediranib 3mg/kg once daily 5 days on 2 days off, Group 4 - olaparib 100mg/kg once daily, Group 5 - cediranib 3mg/kg once daily plus olaparib 100mg/kg once daily, Group 6 - cediranib 3mg/kg once daily 5 days on 2 days off plus olaparib 100mg/kg once daily.

### Example 5: Phase 1 clinical trial results-intermittent dosing of cediranib in combination with MEDI4736 (durvalumab).

The benefit of the intermittent schedule was tested clinically in a combination trial where the ability to combine cediranib with durvalumab was examined using a continuous or an intermittent schedule of cediranib. This trial showed that, while the continuous dose was poorly tolerated, the intermittent schedule was tolerated and resulted in observable clinical benefit. The data are presented below.

### Study Design and Patients

Eligible patients had recurrent or metastatic RECIST v1.1 measurable solid malignancies without prior immune checkpoint inhibitor therapy, controlled hypertension on no more than 3 anti-hypertensives, and good end-organ function; germline *BRCA* mutation status was requested at entry. All patients provided written informed consent before enrollment. The trial was approved by the Institutional Review Board of the Center for Cancer Research, National Cancer Institute. ClinicalTrials.gov identifier: NCT02484404.

Eligible patients received durvalumab+cediranib in a 3+3 dose escalation format according to Table 3. Cohorts enrolled patients simultaneously. Patients were evaluated for toxicity per CTCAEv4. Clinical response was assessed every two cycles by imaging using RECISTv1.1 criteria. Study treatment was discontinued for progression of disease, intercurrent illness, adverse events not recovering to ≤ grade 1 within 14 days, or patient withdrawal of consent.

**Table 3: Dose levels (DL)**

| | **Durvalumab+cediranib** | | |
|---|---|---|---|
| | Cediranib tablets | Durvalumab (MEDI4736), IV | N^{∗} |
| **DL 1** | 20mg | 10mg/kg | 4 |
| | once daily | every 2 weeks | |
| **DL 2** | 30mg | 10mg/kg | 4 |
| | once daily | every 2 weeks | |
| **DL 3** | 20mg (5 days on/ | 1500mg | 6 |
| | 2 days off) | every 4 weeks | |

| | | | |
|---|---|---|---|
| ^{∗} One patient on DL1 withdrew consent on cycle one, one patient on DL2 took cediranib 20mg instead of 30mg for a week during cycle one, and one patient on DL3 developed grade 4 hypertension and off-treatment on cycle one. | | | |

### Definitions of dose-limiting toxicity (DLT) and maximum tolerated dose (MTD)

The primary endpoint of this phase 1 study was to determine recommended phase 2 dose (RP2D) of durvalumab+cediranib combination, defined by the MTD or the highest protocol-defined dose in the absence of DLT. DLT was defined as grade 3 or 4 nonhematologic and grade 4 hematologic adverse events (AEs) related to study medications occurring during the first cycle (28 days). The following were exceptions: grade 3 lymphopenia or leukopenia in the absence of grade 3 or higher neutropenia, grade 3 hypertension controlled with anti-hypertensive therapy, or grade 3 asymptomatic electrolytes imbalance with optimal repletion that downgrades to grade 1 or better within 3 days, grade 3 asymptomatic increase in amylase or lipase that downgrades to grade 1 or better within 7 days after onset of the event, or grade 3 asymptomatic endocrinopathy that is managed with or without systemic corticosteroid therapy and/or hormone replacement therapy. The MTD was defined as the highest dose level at which one or fewer of six patients experienced a DLT. If the observed AE was specifically attributed to only one of the drugs, that drug was held while the patient continued to receive the drug not associated with the observed AE. Treatment-related serious AEs occurring 90 days or more after the last dose of study drugs were reported.

### RESULTS

### Patient characteristics

14 women were enrolled. **Table 4 shows baseline patient characteristics.** Ovarian carcinoma was the most common tumor type (9/14 [64%]).

**Table 4: Baseline characteristics**

| | | | **MEDI4736 (durvalumab) +cediranib (n=14)** |
|---|---|---|---|
| Age (years): median (range) | | | 58.4 |
| | | | (44.4-73.8) |
| Tumor type | | | |
| Ovarian cancer | | | 9 (64%) |
| | platinum-sensitive/platinum-resistant disease | | 4/5 |
| | high-grade serous/clear cell histology | | 7/2 |
| | *BRCA* mutation status^{∗} | | |
| | | germline/wild type/unknown | 2/6/1 |
| | lines of prior therapy | | |
| | | 2-4 | 4 |
| | | ≥5 | 5 |
| | prior PARPi | | 4 |
| | prior bevacizumab | | 6 |
| | ECOG performance status (0/1/2) | | 2/12/0 |
| Cervical cancer, squamous cell histology | | | 2 |
| | lines of prior therapy | | 1, 4 each |
| | prior PARPi | | 0 |
| | prior bevacizumab | | 1 |
| | ECOG performance status (0/1/2) | | 0/2/0 |
| Uterine cancer | | | 3 |
| | endometrial/leiomyosarcoma | | 2/1 |
| | lines of prior therapy | | 1,2,3 each |
| | prior PARPi | | 0 |
| | prior bevacizumab | | 0 |
| | ECOG performance status (0/1/2) | | 1/2/0 |

| | | | |
|---|---|---|---|
| Data are number of patients (total %) or median (range). ECOG=Eastern Cooperative Oncology Group. | | | |

### Dose optimization and toxicities

The recommended phase 2 dose (RP2D) was determined as cediranib 20mg 5 days on/2 days off with durvalumab 1500mg every 4 weeks. Daily cediranib with durvalumab was not well-tolerated, although it did not meet the formal mark of dose-limiting toxicity (DLT) during the first treatment cycle. Daily cediranib was discontinued or dose-reduced due to recurrent grade 2 or non-DLT grade 3 or 4 adverse events (AEs) in 7/8 patients; two patients on DL1 discontinued cediranib due to new pulmonary thromboembolism (PE) on study, and one patient on DL1 was dose reduced on cycle two, with four patients on DL2 dose reduced one level due to recurrent grade 2 abdominal pain, diarrhea, fatigue on later cycles (cycles two, three, and five). Two patients were removed from treatment due to treatment-emergent AE (TEAE) consisting of grade 3 colitis (cycle six) and grade 3 pulmonary hypertension (cycle five). Patients were treated with systemic corticosteroids with no symptom improvement. The patient with pulmonary hypertension also had a PE and expired approximately one month after discontinuation of treatment; autopsy findings revealed disease progression including pericardial effusion, and infiltration of lung, thyroid, lymph nodes, and other organs. A protocol amendment added a new dose level with cediranib 20 mg 5 days on/2 days off. One patient on the intermittent schedule had DLT of grade 4 hypertension on cycle one, and five other patients tolerated the treatment across all administered cycles. All patients had at least one any grade TEAE, summarized in Table 5.

**Table 5: Durvalumab+olaparib and durvalumab+cediranib treatment-related adverse events by maximum grade per patient**

| | **Durvalumab+ daily cediranib (n=8)** | | | | **Durvalumab+ intermittent cediranib (n=6)** | | | |
|---|---|---|---|---|---|---|---|---|
| | Grade 1 | Grade 2 | Grade 3 | Grade 4 | Grade 1 | Grade 2 | Grade 3 | Grade 4 |
| Lymphopenia | 2 | 2 | 1 | 1 | 0 | 0 | 0 | 0 |
| Anemia | 1 | 2 | 2 | 0 | 0 | 0 | 0 | 0 |
| Thrombocytopenia | 6 | 0 | 0 | 0 | 1 | 0 | 0 | 0 |
| Nausea | 0 | 1 | 1 | 0 | 0 | 0 | 0 | 0 |
| Abdominal pain | 1 | 2 | 0 | 0 | 0 | 0 | 0 | 0 |
| Diarrhea | 2 | 2 | 3 | 0 | 3 | 0 | 0 | 0 |
| Anorexia | 1 | 1 | 0 | 0 | 0 | 0 | 0 | 0 |
| Vomiting | 1 | 1 | 0 | 0 | 1 | 0 | 0 | 0 |
| Oral mucositis | 3 | 2 | 0 | 0 | 0 | 0 | 0 | 0 |
| Colitis | 0 | 0 | 1 | 0 | 0 | 0 | 0 | 0 |
| Hypothyroidism | 0 | 2 | 0 | 0 | 0 | 0 | 0 | 0 |
| Hyperthyroidism | 2 | 0 | 0 | 0 | 0 | 1 | 0 | 0 |
| Alkaline phosphatase increased | 2 | 0 | 0 | 0 | 0 | 0 | 0 | 0 |
| AST increased | 2 | 0 | 0 | 0 | 1 | 0 | 0 | 0 |
| ALT increased | 0 | 0 | 0 | 0 | 1 | 0 | 0 | 0 |
| Fatigue | 3 | 2 | 1 | 0 | 2 | 1 | 1 | 0 |
| Headache | 1 | 1 | 1 | 0 | 0 | 0 | 0 | 0 |
| Skin rash | 3 | 0 | 0 | 0 | 1 | 0 | 0 | 0 |
| Pruritus | 0 | 0 | 0 | 0 | 1 | 0 | 0 | 0 |
| Hoarseness | 0 | 0 | 0 | 0 | 1 | 0 | 0 | 0 |
| Weight loss | 1 | 2 | 0 | 0 | 0 | 0 | 0 | 0 |
| Hypertension | 0 | 4 | 3 | 0 | 0 | 4 | 0 | 1 |
| Pulmonary thromboembolism † | 0 | 0 | 1 | 1 | 0 | 0 | 0 | 0 |
| Dyspnea on exertion | 1 | 1 | 0 | 0 | 1 | 0 | 0 | 0 |
| Pulmonary Hypertension † | 0 | 0 | 1 | 0 | 0 | 0 | 0 | 0 |

Data are number. A patient could be counted under more than one preferred term. ALT=alanine aminotransferase. AST=aspartate aminotransferase. One patient on DL1 of durvalumab+daily cediranib withdrew her consent on cycle one day 15 and did not report any AEs. One patient on durvalumab+daily cediranib DL1 had dose reduction to daily cediranib 15mg on cycle two, all four patients on durvalumab+daily cediranib DL2 had dose reduction to daily cediranib 20mg due to recurrent grade 2 abdominal pain, diarrhea, fatigue on cycle two (2 patients), cycle three (1 patient) and cycle five (1 patient). ^{∗}Two patients on durvalumab+daily cediranib received packed RBC transfusion on cycle one (DL1) and cycle five (DL2). †Daily cediranib was discontinued in two patients on DL1 due to pulmonary thromboembolism (PE) on cycle three and cycle five. One with PE developed pulmonary hypertension on cycle five. One patient discontinued daily cediranib and durvalumab due to grade 3 colitis on cycle six.

### Clinical activity

Twelve of 14 patients on durvalumab+cediranib were assessed for tumor response; two were not evaluable due to drug toxicity or withdrawal of consent during cycle one, without demonstrated progression. Six of 12 patients attained a PR (5+-8+ months, 50% ORR), three of those were treated on DL3, suggesting response was not attenuated with the intermittent cediranib schedule. The largest response in the patient's tumor size (from baseline) observed during the trial is shown in Figure 9 (i.e., the largest response observed may have been observed at any time point during the trial). Figure 10 show the changes from baseline in tumor size over time for each patient.

The intermittent cediranib schedule results in improved tolerability and maintained the clinical benefit observed in the daily schedule.

### Example 6: Phase 1 clinical trial-Intermittent dosing of Cediranib in combination with (MEDI4736) durvalumab and olaparib

The benefit of the intermittent schedule was further tested clinically in a combination trial where the ability to combine cediranib with durvalumab and olaparib was examined using an intermittent schedule of cediranib (5 days on/2 days off). The goal of the study was to determine recommended phase 2 dose (RP2D) of durvalumab + olaparib + intermittent cediranib (NCT02484404). This trial showed that intermittent dosing of Cediranib combined with durvalumab and olaparib is tolerable and active in recurrent women's cancers.

### Study design:

Eligible patients with a Performance Status (PS) of 0 to 1 and good end organ function received durvalumab+olaparib+intermittent cediranib. Patients received 15 or 20 mg (5 days on/2 days off) of cediranib with 1500 mg IV every 28 days of durvalumab and with 300 mg tablets of olaparib BID. The dose-limiting toxicity period was one 28 day cycle. Safety was assessed by CTCAEv4.0 and response by RECISTv1.1. All patients provided written informed consent before enrolment.

### Results:

9 women of median age 51 [range 44-73] and median 2 prior therapies [range 2-6] were treated with the durvalumab+olaparib+intermittent Cediranib. 7 patients had ovarian cancer, 1 patient had endometrial cancer and 1 patient had Triple Negative Breast Cancer (TNBC). Two patients experienced grade 3/4 adverse events (lymphopenia). There was no toxicity-related dose reduction or discontinuation. Two partial responses (5⁺, 2⁺ months) and three stable disease (2⁺-7⁺ months) were seen in 5 evaluable patients.

## Claims

1. A composition comprising cediranib for use in a method of treating at least one disease state associated with angiogenesis in a human
wherein said composition is administered for at least two cycles according to a fixed intermittent dosing regimen,
wherein said fixed intermittent dosing regimen consists of administration of an effective amount of said composition over 7 days, wherein said composition is administered on 5 consecutive days followed by 2 days of rest, and
wherein the at least one disease state associated with angiogenesis is chosen from lung cancers; digestive and gastrointestinal cancers; esophageal cancers; gallbladder cancers; liver cancers; pancreatic cancers; appendix cancers; breast cancers; ovarian cancers; renal cancers; cancers of the central nervous system; skin cancers; lymphomas; glioblastomas; choriocarcinomas; alveolar soft part sarcomas; head and neck cancers; osteogenic sarcomas; and blood cancers.

2. The composition for use according to claim 1, wherein the at least one disease state associated with angiogenesis is chosen from platinum sensitive relapsed ovarian cancers.

3. The composition for use according to claim 2, wherein the platinum sensitive relapsed ovarian cancers are chosen from fallopian tube cancers, high grade endometrial cancers, and primary peritoneal cancers.

4. The composition for use according to claim 1, wherein said composition comprises cediranib in an amount of 30 mg or 20 mg as measured by weight of the free base of cediranib.

5. The composition for use according to claim 1, further comprising administering at least one other component chosen from partner drugs and other treatments.

6. The composition for use according to claim 5, wherein said partner drugs are chosen from DNA damage response inhibitors, immune checkpoint inhibitors, tumor cell targeting therapy agents, and chemotherapy agents.

7. The composition for use according to claim 6, wherein said partner drugs are chosen from PARP inhibitors.

8. The composition for use according to claim 7, wherein said PARP inhibitor is olaparib.

9. The composition for use according to claim 6, wherein said partner drugs are chosen from immune checkpoint inhibitors.

10. The composition for use according to claim 9, wherein said immune checkpoint inhibitor is MEDI4736 (durvalumab).

11. The composition for use according to claim 6, wherein said partner drugs are chosen from chemotherapy agents.

12. The composition for use according to claim 11, wherein said chemotherapy agents are chosen from platinum based chemotherapy agents, taxane based chemotherapy agents, and irotecan.

## Patentansprüche

1. Zusammensetzung, umfassend Cediranib, zur Verwendung in einem Verfahren zur Behandlung mindestens eines mit Angiogenese assoziierten Krankheitszustands in einem Menschen,
wobei die Zusammensetzung über mindestens zwei Zyklen gemäß einem fixen intermittierenden Verabreichungsprotokoll verabreicht wird,
wobei das fixe intermittierende Verabreichungsprotokoll aus der Verabreichung einer wirksamen Menge der Zusammensetzung über 7 Tage besteht, wobei die Zusammensetzung an 5 aufeinanderfolgenden Tagen verabreicht wird, gefolgt von 2 Ruhetagen, und
wobei der mindestens eine mit Angiogenese assoziierte Krankheitszustand aus Lungenkrebserkrankungen, gastrointestinalen Krebserkrankungen und Krebserkrankungen des Verdauungstrakts, Speiseröhrenkrebserkrankungen, Gallenblasenkrebserkrankungen, Leberkrebserkrankungen, Bauchspeicheldrüsenkrebserkrankungen, Blinddarmkrebserkrankungen, Brustkrebserkrankungen, Eierstockkrebserkrankungen, Nierenkrebserkrankungen, Krebserkrankungen des zentralen Nervensystems, Hautkrebserkrankungen, Lymphomen, Glioblastomen, Choriokarzinomen, alveolären Weichteilsarkomen, Kopfund Halskrebserkrankungen, osteogenen Sarkomen und Blutkrebserkrankungen ausgewählt ist.

2. Zusammensetzung zur Verwendung nach Anspruch 1, wobei der mindestens eine mit Angiogenese assoziierte Krankheitszustand aus platinsensiblen rezidiven Eierstockkrebserkrankungen ausgewählt ist.

3. Zusammensetzung zur Verwendung nach Anspruch 2, wobei die platinsensiblen rezidiven Eierstockkrebserkrankungen aus Eileiterkrebserkrankungen, hochgradigen Endometriumkrebserkrankungen, und primären Peritonealkrebserkrankungen ausgewählt sind.

4. Zusammensetzung zur Verwendung nach Anspruch 1, wobei die Zusammensetzung Cediranib in einer Menge von 30 mg oder 20 mg, gemessen als Gewicht der freien Base von Cediranib, umfasst.

5. Zusammensetzung zur Verwendung nach Anspruch 1, weiterhin umfassend die Verabreichung mindestens einer anderen aus Partnerarzneimitteln und anderen Behandlungen ausgewählten Komponente.

6. Zusammensetzung zur Verwendung nach Anspruch 5, wobei die Partnerarzneimittel aus Inhibitoren der Reaktion auf DNA-Schädigung, Immuncheckpointinhibitoren, auf Tumorzellen zielenden Therapeutika und Chemotherapeutika ausgewählt sind.

7. Zusammensetzung zur Verwendung nach Anspruch 6, wobei die Partnerarzneimittel aus PARP-Inhibitoren ausgewählt sind.

8. Zusammensetzung zur Verwendung nach Anspruch 7, wobei es sich bei dem PARP-Inhibitor um Olaparib handelt.

9. Zusammensetzung zur Verwendung nach Anspruch 6, wobei die Partnerarzneimittel aus Immuncheckpointinhibitoren ausgewählt sind.

10. Zusammensetzung zur Verwendung nach Anspruch 9, wobei es sich bei dem Immuncheckpointinhibitor um MEDI4736 (Durvalumab) handelt.

11. Zusammensetzung zur Verwendung nach Anspruch 6, wobei die Partnerarzneimittel aus Chemotherapeutika ausgewählt sind.

12. Zusammensetzung zur Verwendung nach Anspruch 11, wobei die Chemotherapeutika aus Chemotherapeutika auf Platinbasis, Chemotherapeutika auf Taxanbasis und Irotecan ausgewählt sind.

## Revendications

1. Composition comprenant du cédiranib destinée à être utilisée dans un procédé de traitement d'au moins un état de maladie associé à une angiogenèse chez un être humain
ladite composition étant administrée pendant au moins deux cycles selon un schéma posologique intermittent fixé,
dans laquelle ledit schéma posologique intermittent fixé est constitué par une administration d'une quantité efficace de ladite composition pendant 7 jours, ladite composition étant administrée sur 5 jours consécutifs suivis de 2 jours de repos, et
dans laquelle l'au moins un état de maladie associé à une angiogenèse est choisi parmi des cancers du poumon ; des cancers digestifs et gastrointestinaux ; des cancers de l'œsophage ; des cancers de la vésicule biliaire ; des cancers du foie ; des cancers du pancréas ; des cancers de l'appendice ; des cancers du sein ; des cancers de l'ovaire ; des cancers du rein ; des cancers du système nerveux central ; des cancers de la peau ; des lymphomes ; des glioblastomes ; des choriocarcinomes ; des sarcomes alvéolaires des parties molles ; des cancers de la tête et du cou ; des sarcomes ostéogéniques ; et des cancers du sang.

2. Composition destinée à être utilisée selon la revendication 1, dans laquelle l'au moins un état de maladie associé à une angiogenèse est choisi parmi des cancers de l'ovaire récidivants sensibles au platine.

3. Composition destinée à être utilisée selon la revendication 2, dans laquelle les cancers de l'ovaire récidivants sensibles au platine sont choisis parmi des cancers des trompes de Fallope, des cancers de l'endomètre de haut grade et des cancers péritonéaux primitifs.

4. Composition destinée à être utilisée selon la revendication 1, ladite composition comprenant du cédiranib en une quantité de 30 mg ou de 20 mg, tel que mesuré en poids de la base libre de cédiranib.

5. Composition destinée à être utilisée selon la revendication 1, comprenant en outre une administration d'au moins un autre composant choisi parmi des substances médicamenteuses partenaires et d'autres traitements.

6. Composition destinée à être utilisée selon la revendication 5, dans laquelle lesdites substances médicamenteuses partenaires sont choisies parmi des inhibiteurs de la réponse à un endommagement de l'ADN, des inhibiteurs de points de contrôle immunologiques, des agents de thérapie de ciblage de cellules tumorales et des agents de chimiothérapie.

7. Composition destinée à être utilisée selon la revendication 6, dans laquelle lesdites substances médicamenteuses partenaires sont choisies parmi des inhibiteurs de PARP.

8. Composition destinée à être utilisée selon la revendication 7, dans laquelle ledit inhibiteur de PARP est l'olaparib.

9. Composition destinée à être utilisée selon la revendication 6, dans laquelle lesdites substances médicamenteuses partenaires sont choisies parmi des inhibiteurs de points de contrôle immunologiques.

10. Composition destinée à être utilisée selon la revendication 9, dans laquelle ledit inhibiteur de point de contrôle immunologique est le MEDI4736 (durvalumab).

11. Composition destinée à être utilisée selon la revendication 6, dans laquelle lesdites substances médicamenteuses partenaires sont choisies parmi des agents de chimiothérapie.

12. Composition destinée à être utilisée selon la revendication 11, dans laquelle lesdits agents de chimiothérapie sont choisis parmi des agents de chimiothérapie à base de platine, des agents de chimiothérapie à base de taxanes et l'irotécan.


### SEQUENCE LISTING

<110> AstraZeneca AB National Institutes of Health
<120> METHODS COMPRISING FIXED INTERMITTENT DOSING OF CEDIRANIB
<130> 09963.0087-00304
<150> 62/295421
   <151> 2016-02-15
<160> 8
<170> PatentIn version 3.5
<210> 1
   <211> 108
   <212> PRT
   <213> Homo sapiens
<400> 1
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Arg Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Asp Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Leu Pro
                   85                  90                  95
   Trp Thr Phe Gly Gln Gly Thr Lys Val Glu Ile Lys
               100                 105
<210> 2
   <211> 121
   <212> **PRT**
   <213> Homo sapiens
<400> 2
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Arg Tyr
                   20                  25                  30
   Trp Met Ser Trp Val Arg Gln Ala Pro Gly Lys Gly Leu Glu Trp Val
           35                  40                  45
   Ala Asn Ile Lys Gln Asp Gly Ser Glu Lys Tyr Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Gly Trp Phe Gly Glu Leu Ala Phe Asp Tyr Trp Gly
               100                 105                 110
   Gln Gly Thr Leu Val Thr Val Ser Ser
           115                 120
<210> 3
   <211> 5
   <212> **PRT**
   <213> Homo sapiens
<400> 3
   Arg Tyr Trp Met Ser
   1               5
<210> 4
   <211> 17
   <212> **PRT**
   <213> Homo sapiens
<400> 4
   Asn Ile Lys Gln Asp Gly Ser Glu Lys Tyr Tyr Val Asp Ser Val Lys
   1               5                   10                  15
   Gly
<210> 5
   <211> 12
   <212> **PRT**
   <213> Homo sapiens
<400> 5
   Glu Gly Gly Trp Phe Gly Glu Leu Ala Phe Asp Tyr
   1               5                   10
<210> 6
   <211> 12
   <212> **PRT**
   <213> Homo sapiens
<400> 6
   Arg Ala Ser Gln Arg Val Ser Ser Ser Tyr Leu Ala
   1               5                   10
<210> 7
   <211> 7
   <212> PRT
   <213> Homo sapiens
<400> 7
   Asp Ala Ser Ser Arg Ala Thr
   1               5
<210> 8
   <211> 9
   <212> **PRT**
   <213> Homo sapiens
<400> 8
   Gln Gln Tyr Gly Ser Leu Pro Trp Thr
   1               5